# EUROPEAN PATENT SPECIFICATION

(11) **EP 1 701 715 B1**
(45) Date of publication and mention of the grant of the patent: **05.10.2016**
(21) Application number: 04815753.1
(22) Date of filing: 23.12.2004
(51) Int. Cl.: A61K 31/065, A61P 27/00, A61K 31/047, A61K 31/07, A61K 9/00, A61K 31/355, A61K 31/375, A61K 33/30, A23L 33/10

(54) **ZEAXANTHIN FORMULATIONS WITH ADDITIONAL OCULAR-ACTIVE NUTRIENTS, FOR PROTECTING EYE HEALTH AND TREATING EYE DISORDERS**
ZEAXANTHIN-FORMULIERUNGEN MIT ZUSÄTZLICHEN AUGENAKTIVEN NÄHRSTOFFEN ZUM SCHUTZ DER AUGENGESUNDHEIT UND ZUR BEHANDLUNG VON AUGENERKRANKUNGEN
FORMULATIONS DE ZEAXANTHINE AVEC NUTRIMENTS D'ACTIVITE OCULAIRE ADDITIONNELS, POUR LA PROTECTION DE LA SANTE OCULAIRE ET LE TRAITEMENT DE TROUBLES OCULAIRES

(30) Priority: 23.12.2003 US 746403
(43) Date of publication of application: 20.09.2006
(73) Proprietor: ZeaVision, LLC, Chesterfield, MO 63017 (US)
(72) Inventor: GIERHART, Dennis, L., Chesterfield, MO 63017 (US)
(74) Representative: Grünecker Patent- und Rechtsanwälte PartG mbB
(86) International application number: PCT/US2004/043743
(87) International publication number: WO 2005/063223

(56) References cited:
- WO-A-02/47493
- US-A1- 2001 009 926
- US-A1- 2001 031 744
- US-B1- 6 573 299
- US-B1- 6 649 195

## Description

### BACKGROUND OF THE INVENTION

This invention is in the fields of biochemistry, pharmacology, and nutritional supplements, and relates to orally-ingested formulations for treating or preventing eye diseases and vision problems.

Hundreds of different dietary supplements, under thousands of brand and product names, are being marketed to the public in the U. S. and elsewhere, by means of advertising promises and claims suggesting that these products can help prevent or treat eye diseases, and maintain eye health. Faced with an overwhelming glut of competing promises and products, nearly all of which are unproven and many of which have only tenuous and flimsy support, it has become effectively impossible for people who are concerned about eye health to know which products will help, and which are merely preying on innocent victims whose vision is deteriorating, either because of general aging problems, or due to specific diseases, infections or injuries.

Those uncertainties and doubts extend to full-time professionals who specialize in eye research, or in treating eye diseases. Even the most highly skilled optometrists and ophthalmologists simply do not know whether even the most well-known and widely-used nutritional supplements are actually safe and effective, in treating various eye diseases.

Numerous articles (including several cited below) that have been published in respected scientific and medical journals support this assertion, and state that not enough evidence is available to allow physicians to know whether to recommend various candidate dietary supplements to their patients.

That assertion can also be supported, and brought into better focus, by considering the outcomes and results of the so-called"Age-Related Eye Disease Study" (AREDS). As of late 2004, the benchmark"standard of care"for treating macular degeneration are two nearly identical products that arose from the AREDS trial. Those two products are being sold under the trademarks OCUVITE PRESERVISION by Bausch & Lomb, and I-CAPS AREDS by Alcon Laboratories.

Since those products, and the AREDS trial, have established the "standard of care" for treating or preventing macular degeneration, they are discussed in more detail in the following section.

### THE AREDS TRIAL, AND AREDS PRODUCTS

The AREDS trial was a multi-site human clinical trial involving thousands of elderly patients. It was organized and conducted, at a cost of tens of millions of dollars, by the National Eye Institute (unless otherwise noted, all governmental agencies referred to herein are in the United States of America).

Some of the elderly people who participated in one "treatment arm" of the study received three anti-oxidant vitamins, which were Vitamin A (in the form of beta-carotene), Vitamin C (ascorbic acid) and Vitamin E (alpha-tocopherol), all at high dosages. Those vitamins, by themselves, did not offer any statistically significant benefits, in any categories of patients. In a second "treatment arm" of the study, other elderly people were given heavy dosages (80 mg/day of elemental zinc, in the form of zinc oxide). The zinc by itself also did not offer any statistically significant benefits, in any categories of patients. In a third "treatment arm," still other elderly people were given Vitamins A, C and E, as well as zinc.

In all three treatment arms, participants were divided into four categories, depending on their eye health when they entered the study. Category 1 was at the low end of the scale, and contained participants who had no apparent signs of age-related macular abnormalities. Category 4 was the high end of the scale, and contained participants who suffered from serious macular problems in one eye, while the other eye remained sufficiently free of advanced problems to allow useful monitoring and measuring of any subsequent declines, after the person began taking the supplements.

Some benefits were seen from the combined vitamins-plus-zinc treatment, but only in the subcategory 3 and 4 patients in that treatment arm. Detailed information on the macular degeneration is available in AREDS Report #8 (Archives of Ophthalmology 119: 1417-1436 (October 2001). Additional results on cataracts are described in AREDS Report #9, which immediately follows Report #8 in the same journal.

Based on the macular benefits that were seen in Category 3 and 4 patients in the vitamins-plus-zinc treatment arm of the AREDS test, the managers of the study reported that they had found the first effective treatment for macular degeneration, and Bausch & Lomb (which had provided partial funding for the study) began advertising that its OCUVITE PRESERVISION product was the first "clinically proven" treatment that could help prevent and treat macular degeneration. Using the results of the AREDS trial in various affidavits (which are available for public inspection in the file wrapper of the patent application), Bausch & Lomb obtained US patent 6,660,297 (Bartels et al) in December 2003. It filed a patent infringement lawsuit against Alcon's I-CAPS AREDS product on the same day the patent was issued, in federal court in Rochester, New York. The proceedings of that lawsuit are open for public inspection, under docket 6:03-CV-06617.

Returning to the AREDS trial, a number of commentators, and certain factors that have drawn closer attention since October 2001, have raised questions and concerns over both the safety and efficacy of the AREDS products that are being sold to elderly consumers. The negative commentaries and concerns include the following:
1. A number of skilled researchers and physicians have challenged the findings of the study. As one example, Siegel 2002 publicly and openly complained, in a letter to the editor entitled, "AREDS Investigators Distort Findings," that any claimed benefits of the combined antioxidants-plus-zinc treatment could be teased out of the data only by massaging the data in ways that, instead of being objective, impartial, and scientific, were instead biased, and intended to locate something positive to report, in order to offset the fact that the entire remainder of the study had spent many millions of dollars but had come up empty. In the words of that expert, "In my opinion the AREDS investigators promoted a nonsignificant result into a conclusive recommendation. Here is how they did it.... the message that should have emerged from AREDS is that these treatments failed to demonstrate efficacy in preventing AMD and are not recommended for that use."
2. Even reviewers who endorsed the AREDS findings had to include cautions and caveats. As one example, Jampol 2001, an editorial that accompanied the main AREDS report in the same issue of the same journal, included statements such as, "The exclusion of the subgroup of patients in Category 2 from many of the analyses because of the low incidence of primary outcome events in troubling because it came after review of the data. " Two years later, in Jampol 2003, that same author published an update, which contained statements such as, "The potential toxicity of both the AREDS formulation and these other preparations should be kept in mind.... Evidence supports the use of the AREDS supplements only in patients with intermediate or advanced AMD. "
3. It has also become clear that heavy dosages of beta-carotene in the AREDS formulations almost certainly are not providing any significant benefits for eye health, and instead are creating substantial and even lethal risks among some segments of the elderly population, including smokers. It has been known for years that, although beta-carotene is an anti-oxidant at relatively low oxygen concentrations, it actually takes on the opposite role, and becomes a ***pro***-oxidant (i.e., an agent that triggers and increases the formation of destructive oxygen radicals) at high oxygen concentrations. Those high oxygen concentrations do not exist in most tissues of the body; however, they do exist in the lungs, which directly interact with oxygen in air that is being breathed. Therefore, large and reliable studies in several nations have clearly shown that high dosages of beta-carotene increase the risks and rates of lung cancer among smokers. This is an important concern, since smoking increases the risks of macular degeneration, and elderly smokers comprise an important segment of the population that is at elevated risk of macular degeneration.
4. In addition, other studies have shown that high dosages of Vitamin A and beta-carotene are associated with decreased bone density, and elevated risks of bone fractures. Clearly, such bone-related risks are of major concern among the elderly.
3. Questions also have been raised about the safety of abnormally high dosages of Vitamins C and E. For example, studies have indicated that post-menopausal women who suffer from coronary artery disease should not take high dosages of vitamins C or E. At first glance, that might seem like a relatively small subcategory of people who should be disqualified from taking AREDS supplements; however, it actually is a large and important subset of people who are concerned about macular degeneration. Age-related macular degeneration doesn't become a significant concern until people reach their 60's, 70's, and 80's; therefore, nearly any woman who must decide whether to take an AREDS supplement will indeed be of post-menopausal age. After that factor has been recognized, it must then be recognized that nearly everyone who has reached that age has some level of plaque buildup in their arteries, especially if they live in an industrialized country where food intake over the course of a lifetime includes substantial quantities of fatty foods (this factor deserves attention, since fatty foods simultaneously increase both the risks of arterial plaques, and the risks of macular degeneration, thereby creating a causative link and overlap between those two problems). Therefore, serious questions must be asked about whether, and under what conditions, elderly women (especially elderly women who may be a few pounds overweight, and/or who have high cholesterol levels) can safely take extra-heavy dosages of vitamins C and E.

These and similar concerns about widespread, uncontrolled, and potentially dangerous intake of high-dosage vitamins are discussed in items such as Gaynes 2003 and Pulido et al 2004 (which relate specifically to the AREDS products that currently are being sold to elderly people), and in numerous other articles that discuss general concerns about heavy vitamin intake (often referred to as "mega-vitamin" intake).
4. Finally, major questions have been raised about whether extra-heavy dosages of zinc will create or aggravate risks involving neurotoxicity, Alzheimer's disease, and brain damage. Because of their importance, those concerns are addressed in the next section.

### NEUROTOXIC RISKS OF HEAVY ZINC INTAKE

The OCUVITE PRESERVISION supplements (sold by Bausch & Lomb) and the I-CAPS AREDS supplements (sold by Alcon Laboratories) are labelled and advertised as containing daily dosages of 69.6 milligrams per day of elemental zinc. These zinc dosages are nearly nine times higher than the "Recommended Daily Allowance" (RDA) levels for women, and more than six times higher than the RDA levels for men. Just as importantly, these levels are nearly twice as high as the "Tolerable Upper Intake Levels" for zinc that were established in 2001, by impartial experts working for the Institute of Medicine, which is part of the National Academy of Sciences. It should also be noted that neither of the products named above can be sold legally in Europe or most other countries, because of their heavy zinc dosages.

None of the first 11 published reports on the AREDS trial recognized, addressed, or cited even a single one of the numerous articles listed below, which raise major concerns about the risk of neurotoxicity and brain damage, when extra-heavy zinc dosages are taken by elderly people. Since elderly people are indeed the target population and the target market for any product that is sold and used to prevent or treat macular degeneration, these neurotoxicity risks merit serious concern, and careful consideration. However, these concerns apparently have been ignored or dismissed, by the managers who organized the AREDS trial, and by the consultants who authored the reports which describe and analyze the data generated by that trial.

One concern involves the role of zinc in accelerating the formation and growth of beta-amyloid plaques, which are crucially important in Alzheimer's disease, a devastating disease that destroys the brains and minds of elderly victims. The linkage between zinc, beta-amyloid plaque formation and growth, and Alzheimer's disease, is described in a series of articles that include Bush et al 1994, Cherny et al 2001, Ritchie et al 2003, Bush 2003, Finefrock et al 2003, and Cuajungco et al 2003.

The second major neurotoxicity concern involves the risk that heavy zinc intake may increase the severity and extent of permanent brain damage and impairment, if an elderly person suffers a stroke, cardiac arrest, or other crisis involving ischemia (inadequate blood flow) or hypoxia (inadequate oxygen supply). Reports describing this risk include Choi et al 1998, Lee et al 2002, Sheline et al 2003, Canzoniero et al 2003, Ryu et al 2002, Manzerra et al 2001, Lobner et al 2000, Suh et al 2000, and numerous other reports cited therein.

When the AREDS trial was first being planned and organized in the late 1980's and early 1990's, those neurotoxicity concerns were not known; they had not been discovered or reported, by anyone. Therefore, the people who planned and conducted the AREDS trial are blameless for deciding to include 80 mg/day dosages of zinc in the AREDS trials, since that dosage had been previously tested and reported to do at least some good in slowing down macular degeneration in at least some patients.

However, questions arise about how the neurotoxicity concerns have been handled by managers and consultants working on the AREDS project since about 2000, since a number of reports that raised serious warning signals had been published by then, and can be easily located in numerous databases, including the free database offered by the National Library of Medicine in the U.S. Instead of facing up to those concerns, the only AREDS items published to date on zinc's effects on the brain have sidestepped the issues, in ways that create more questions than answers.

Clemons et al 2002 is a published abstract entitled, "Cognitive function in the Age-Related Eye Disease Study (AREDS)". It reported a reassuring conclusion that no adverse impacts on brain functioning were seen in the AREDS data. However, that conclusion was based on an arbitrary and questionable standard, summarized by the following sentence from the abstract: "Cognitive impairment and depression, ***defined as falling below the 10th percentile of the AREDS population,*** was assessed using logistic regression" [emphasis added].

That sentence indicates that if someone dropped from a 90% level of performance, down to a 15 or 20% level of performance, that steep decline would actually be classified and treated as a positive and reassuring outcome, based on the arbitrary criterion that someone had to fall all the way to the lowest 10% of the test population before an effect would be recognized and classified as negative. Clearly, any impartial observer would sense that a drop from a performance level in the top half or quarter of the population, down to the lowest 15 or 20%, would indicate that something bad had happened; however, under the arbitrary standard quoted above, that steep decline would be reported not just as a neutral outcome, but as a positive, favorable, and reassuring outcome.

More information was contained in AREDS Report #12 (Yaffe et al 2004), which claimed that its results were "reassuring" that zinc was not having any adverse impacts on mental functioning. However, an analysis of that report reveals serious gaps and unanswered questions. For example, the data were based solely on people who completed a battery of mental tests, at the end of their participation in the AREDS trial; however, it did not say whether any mental testing was done near the start of the trial. A different abstract published separately, which disclosed that the progression and severity of macular degeneration correlated with broader mental deterioration, indicated that cognitive testing over at least a 2-year span was in fact done on many participants; however, the authors of Yaffe et al 2004 did not disclose that testing, and did not disclose any data from those tests, in their article.

It must also be pointed out that 40% of the people who completed the AREDS trial did not complete the mental tests, and were excluded from the analysis that led to the reassurances by Yaffe et al. That raises a major question and concern. If high zinc concentrations may trigger or accelerate Alzheimer's disease or other neurotoxic problems, as suggested by the reports cited above, then people who may have been adversely affected likely would not be able to complete the entire battery of mental tests that were given at the end of the trial; and, if that subset of people could not complete those tests, they were simply excluded from the data-gathering, and from the analysis. Clearly, if people who may have been adversely affected by heavy zinc intake were simply pushed off to one side and excluded from any analysis of the remaining data, that raises questions, since it would strongly bias any interpretations and conclusions toward a positive outcome, when the actual facts might indicate serious problems. However, that factor was not mentioned or addressed in Yaffe et al 2004.

In addition, although Yaffe et al 2004 cited the Bush et al 1994 article on zinc and Alzheimer's disease, it did not cite or consider any of the articles that have shed more light on the Alzheimer's linkage since 1994. Just as importantly, it did not cite or consider any articles describing apparent links between high zinc levels, and aggravated brain damage after a stroke or other crisis. It also it did not cite or address other articles such as Linkous et al 2004, which reported that in laboratory animals, dietary intake of excess zinc led to elevated zinc levels in brain tissue, and to impaired mental performance in those animals.

In addition to the neurotoxicity risks cited above, intake of extra-heavy dosages of zinc may also lead to other serious health problems. For example, Leitzmann et al 2003 describes the largest and most powerful study ever done of a possible link between heavy zinc intake, and prostate cancer. In a survey of nearly 50,000 male health-care professionals over 14 years, men who ingested large dosages of zinc suffered from increased rates of prostate cancer.

Finally, it should be noted that in 2001, the Institute of Medicine revised its "Dietary Reference Intake" numbers for zinc. In one change, it reduced the "Recommended Daily Allowance" (RDA) numbers for adults from pre-2001 levels of 15 mg/day, to 8 mg/day for females, and 11 milligrams per day for males. However, even though that change in the RDA numbers was made several years ago, the labeling and advertising used by both Bausch & Lomb and Alcon continues to rely on the old and outdated pre-2001 RDA number. As a result, both companies describe their products as containing 464% of the RDA for zinc. However, using current and accurate RDA numbers, the products being sold by those two companies actually contain 870 % of the RDA for women, and 633 % of the RDA for men.

Even more importantly, the Institute of Medicine also issued a "Tolerable Upper Intake Level" for zinc in 2001. That number is 40 mg/day for all adults, regardless of gender or age. Therefore, the zinc levels in OCUVITE PRESERVISION and I-CAPS AREDS products are nearly twice as high as the levels declared by experts to be the "Tolerable Upper Intake" levels. The surplus becomes even higher if dietary zinc is also considered, and it becomes higher still, among elderly people who are also taking multi-vitamins or other "nutritional supplements" that also contain zinc.

Finally, it should be noted that Bausch & Lomb and Alcon added copper to their OCUVITE PRESERVISION and I-CAPS AREDS products, to balance out a competitive interaction between zinc and copper, involving digestive uptake. However, copper poses a risk of aggravating the severity of brain damage in people who suffer from Alzheimer's disease, since it catalyzes the formation of peroxide compounds and oxidative radicals, in beta-amyloid plaques. Therefore, the addition of copper, to formulations containing heavy zinc dosages being taken by elderly consumers, may solve one problem, but it may also create or aggravate a different problem, especially in people who have incipient, early, or mid-stage Alzheimer's disease.

The important point to recognize and understand, from the foregoing summary of concerns, is this: despite all of these concerns, risk factors, and unanswered questions, the AREDS formulations have created, established, and defined the current best-known standard of care, for elderly people who suffer from or who are concerned about macular degeneration. The OCUVITE PRESERVISION product is the most widely known, widely used, and widely recommended nutritional supplement available today, for treating or preventing macular degeneration.

Or, more accurately, it is the most widely known, used, and recommended supplement available in the United States. It cannot be sold or used legally throughout most of Europe and in many other countries, mainly because its heavy daily dosage of zinc has not been proven safe, to the satisfaction of the authorities in various nations.

The bottom line is that the current best "standard of care" that is known to ophthalmologists and other health care professionals falls short of being ideal, or even adequate. It is not very effective, it creates potentially serious health and neurological risks that have not been adequately addressed, and expert reviewers who have studied the AREDS trial carefully have concluded that the AREDS supplements should be recommended by physicians "only in patients with intermediate or advanced AMD" (Jampol 2003), and should not be recommended to, or taken by, elderly people who do not suffer from intermediate or advanced macular degeneration.

Accordingly, this current invention needs to be evaluated by comparing it to the current best "standard of care" that is known to, and recommended by, skilled ophthalmologists and physicians. Despite the risks listed above, and despite its limited efficacy, the current "standard of care" is embodied by the Bausch & Lomb OCUVITE PRESERVISION product, which dominates the market (at least in the US), and which is advertised as the only nutritional supplement that has been "clinically proven" to help treat or prevent macular degeneration.

### CAROTENOIDS IN THE RETINA: BETA-CAROTENE, LUTEIN, AND ZEAXANTHIN

Carotenoids are another important class of compounds that need to be considered, in any effort to develop nutritional supplements that can prevent or treat macular degeneration.

Carotenoids were given that name because they were first isolated from carrots. They evolved in plants and in some types of bacteria, over millions of years, because they can absorb ultraviolet (UV) radiation without being damaged. Since UV radiation is toxic and even lethal to cells that are exposed to prolonged sunlight, carotenoids became crucially important to the survival of plants and microbes that gradually moved from submerged marine environments, to exposed land environments.

Animals cannot synthesize carotenoids in their bodies. Therefore, they must ingest carotenoids as part of their diets. In this regard, carotenoids are directly comparable to vitamins, and can be considered as a class of vitamins.

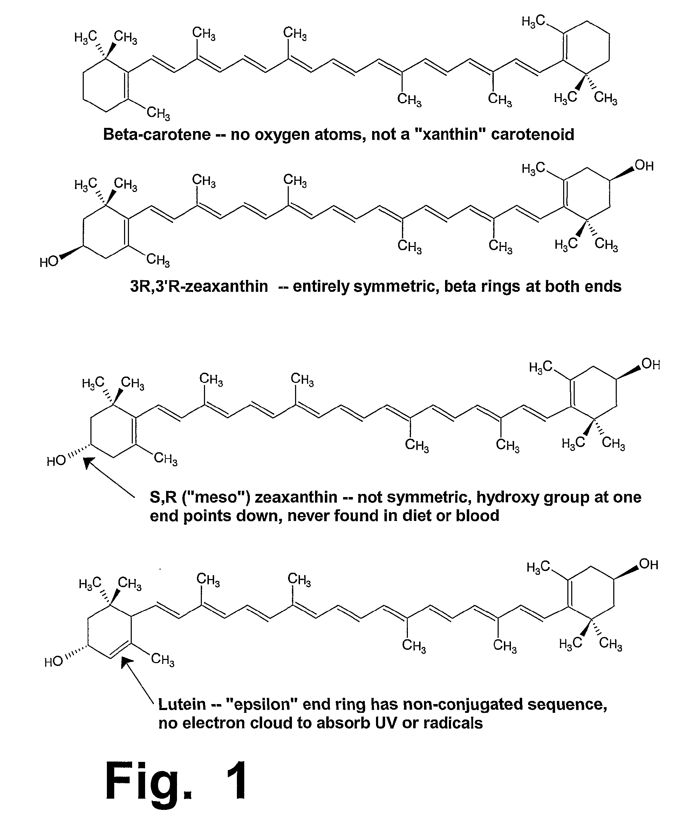

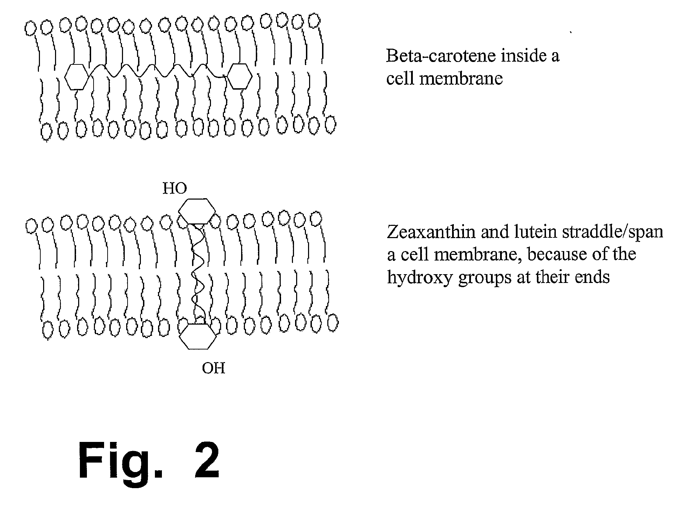

The structures of several carotenoids that are relevant to eye care formulations are shown in FIG. 1 (which is prior art, since these molecular structures have been known for years). Several specific factors in these molecular structures are worth noting, as follows:
1. In the straight chain portion (between the two "end rings") of each carotenoid shown in FIG. 1, the double bonds alternate with single bonds. That pattern of alternating single and double bonds is called "conjugation". It is important, because when a series of single and double bonds are conjugated, the electrons that form bonds between adjacent atoms do not remain attached to specific atoms. Instead, the electrons become mobile, and they form an "electron cloud" that covers and surrounds the molecule. This same type of electron cloud also surrounds and stabilizes benzene rings and other aromatic molecules.
2. The conjugated electron cloud that surrounds parts of a carotenoid molecule is important, because it leads to remarkable results. First, when a carotenoid molecule is hit by ultraviolet light, the carotenoid will not break. Instead, the electron cloud is able to flex and yield, in a way that cushions and absorbs the blow. This is comparable to someone hitting wooden board and a rubber tire, with a sledgehammer. The board will break, because it cannot bend or deflect. However, a rubber tire will not break, because it can flex and yield in a way that allows it to absorb the force of the blow. When a UV photon hits a carotenoid molecule, the destructive power of that photon is used up and absorbed by the conjugated electron cloud. This prevents the UV photon from attacking and damaging other crucial molecules, such as strands of protein or DNA. By absorbing UV radiation, carotenoids protect DNA, proteins, lipids that form cell membranes, and other crucially important molecules in cells.
3. In addition to protecting molecules and cells against UV radiation, the conjugated electron cloud around a carotenoid molecule can also absorb, neutralize, and "quench" oxygen-containing radicals. These radicals (also called "reactive oxygen species" (ROS) and similar terms) are unstable, aggressive, and destructive, due to unpaired electrons in their valence shells.
   The ability to neutralize oxygen radicals is called anti-oxidant activity, and under most conditions, carotenoids are useful anti-oxidants. However, as mentioned above in the discussion of increased risks of lung cancer in smokers, under some conditions, some carotenoids (including beta-carotene) can reverse their anti-oxidant activity and become pro-oxidants (i.e., agents that accelerate the formation of destructive oxygen radicals).
4. Referring again to the structures in FIG. 1, zeaxanthin and lutein have "hydroxy" (HO-) groups attached to their end rings, while beta-carotene has no oxygen atoms or hydroxy groups. This leads to crucial differences in the way they are deposited and positioned in animal cell membranes, after they are eaten by animals. Since beta-carotene is made entirely of hydrocarbons, with no hydroxy groups, it is non-polar, which makes it soluble in oily liquids made only of hydrocarbons. Therefore, it will position itself in an animal cell membrane in the manner shown in FIG. 2, which depicts a beta-carotene molecule aligned in a manner that is roughly parallel to the surface of a cell membrane, sitting entirely within the center of the membrane. This results from the fact that animal cell membranes are made up of "bilayers" formed by molecules called phospho-lipids. These molecules will spontaneously line up together, when placed in a watery fluid, in a way that gives them a bilayer arrangement as shown in FIG. 2. Each phospho-lipid molecule has a polar (and therefore hydrophilic and water-soluble) "head" that contains phosphorous, bonded to a lipid (and therefore hydrophobic) "tail" made of hydrocarbons. Therefore, when phospho-lipid molecules form membranes, they create an oily hydrophobic layer (with the hydrocarbon tails) in the middle of the membrane, surrounded by hydrophilic phosphate heads on both surfaces of the membrane.

As a result, as shown in FIG. 2, beta-carotene (an oily hydrocarbon with no oxygen atoms or hydroxy groups) will be deposited in the oily center of a cell membrane. By contrast, zeaxanthin and lutein (which have hydroxy groups on both end rings) will be deposited in a manner that causes them to "span" or "straddle" the membrane, with their end rings protruding slightly beyond the inner and outer surfaces of the membrane (because plants and animals evolved cooperatively, it is not just a coincidence that zeaxanthin and lutein are just long enough to span the thickness of animal cell membranes, with portions of their end rings sticking out from both sides of the membrane).

The most common fate of beta-carotene is that it is split in half, by enzymes, to release two molecules of a compound called retinol, also known as Vitamin A. The cleavage process involves hydrolysis, and it adds a hydroxy group to the end of the straight-chain portion of the compound that becomes retinol (Vitamin A). Retinol is converted by other enzymes into a similar compound called retinal, and certain straight-chain and bent-chain isomers of retinal are essential in the chemical reactions that enable rod and cone receptors (which are components of retinal neurons) to convert incoming light into nerve signals, which emerge from the retina and travel to the brain. Therefore, beta-carotene and Vitamin A are essential to vision, since they supply the precursors to the isomers of retinal that are crucial to rod and cone receptors.

Accordingly, beta-carotene plays an essential role in vision, and since it is also known to be an anti-oxidant, it was a good candidate for testing, to see whether high doses of beta-carotene could help prevent or treat macular degeneration. Therefore, high-dosage beta-carotene was indeed included, in the AREDS trial.

However, the results of the AREDS trial made it clear that beta-carotene, even when combined with Vitamin C and Vitamin E, did not provide any significant benefits against macular degeneration. By the late 1990's, it became clear for a number of reasons that high dosages of beta-carotene or Vitamin A would offer little or no serious hope for providing any significant benefits against macular degeneration, or any other serious eye disorders, among people who receive adequate baseline levels of vitamin A. Therefore, in the patent application that eventually issued as US 6,660,297 (Bartels et al 2003), the inventors specifically stated that beta-carotene could be omitted from the formulation, and replaced by lutein and/or zeaxanthin.

Lutein and zeaxanthin are of direct and special interest in efforts to prevent macular degeneration, because it has been known since the publication of Bone et al 1985 that those are the two carotenoids that give the macula its distinctive yellowish color. Several dietary surveys (notably including Seddon et al 1994) have indicated that diets rich in dark green vegetables (which contain lutein and zeaxanthin) appear to reduce the risks of macular degeneration.

However, after the Seddon report was published in 1994 and provoked increased interest in the field, it triggered a followup analysis, which involved blood samples that had been previously gathered from thousands of elderly people, in the so-called "Beaver Dam Study" (named after Beaver Dam, Wisconsin, a town that was chosen as the subject of a large multi-year study of eye health involving thousands of people). The results, published in Mares-Perlman et al 1995, were based on chemical testing of blood concentrations of lutein, zeaxanthin, and other carotenoids (which, it must be noted, are much more reliable than memories and potentially biased, self-justifying answers to questions that are asked in a dietary survey). The data and conclusions published in Mares-Perlman et al 1995 directly contradicted the conclusions of the Seddon 1994 report. In particular, that study clearly and directly stated, "Levels of the carotenoids that compose macular pigment (lutein with zeaxanthin) in the [blood] serum were unrelated to ARMD [age-related macular degeneration]."

In view of those conflicting reports and other problems, experts in eye research, and ophthalmologists who specialize in treating patients with serious eye problems, do not and cannot agree on the roles or potential benefits of lutein and/or zeaxanthin, in the retina. Evidence to support and prove this conclusion is available from numerous sources, both published and unpublished. Examples of such published reports by experts include Schalch 2000, and Jampol 2001.

Wolfgang Schalch is one of the foremost researchers in the world, in the field of carotenoids. For years, he has been one of the top carotenoid research managers at Roche Vitamins, the world's largest manufacturer and supplier of carotenoids (it should be noted that Roche Vitamins, a division of the Hoffman-LaRoche pharmaceutical company, was sold to DSM Chemicals in 2003). In a 2000 article, he distinguished between epidemiological studies (which look for statistical correlations in populations based on past events, such as Seddon's dietary survey, and Mares-Perlman's blood sample analyses) versus intervention studies (which begin with populations that are divided into subgroups, which are then fed different dietary levels of compounds, and which are monitored for a period of time to see what effects the dietary differences have on the different subgroups).

Schalch 2000, at page 38, describes the serious problems that hinder and limit epidemiological studies, but then he also notes the difficulties that will arise if someone tries to carry out intervention studies using lutein and zeaxanthin. In Schalch's words, "Epidemiological studies therefore cannot provide definite proof of the efficacy of lutein and zeaxanthin in AMD. Such studies can provide evidence of possible relationships but cannot determine whether an effect is causal. The situation is different with intervention studies in which agents are administered on a double-masked, placebo-controlled, randomized basis and results are evaluated using predefined efficacy parameters. In the case of supplementation witn lutein and zeaxanthin, where only small to moderate responses can be expected, only studies such as these [i.e., intervention studies] are likely to provide a definite answer as to an effect of lutein and zeaxanthin on AMD. However, the specific time-course and nature of this disease makes the design of such trials difficult."

Lee Jampol is another world-class expert. He was chosen by the editors of the highly respected journal, Archives of Ophthalmology (a journal published by the American Medical Association) to sort through the large amounts of data in the AREDS reports, and summarize and explain those findings to other physicians. Accordingly, Jampol wrote the commentary that accompanied and prefaced the two main AREDS reports (AREDS report number 8, on macular degeneration, and AREDS report number 9, on cataracts, both of which appeared in the October 2001 issue of *Archives of Ophthalmology,* In his 2001 analysis and commentary, Jampol stated on page 1534, "In view of previous studies suggesting that beta-carotene might be harmful in smokers and may be associated with a greater risk of lung cancer, beta-carotene should probably not be used by smokers and recent ex-smokers. An argument could be made that another carotenoid, lutein or zeaxanthin, could be substituted for beta-carotene, but the values and risks of other carotenoids [referring to lutein and zeaxanthin] is unknown at this time."

Two years later, in November 2003, Jampol updated his analysis and commentary. In a commentary entitled, "AREDS - Two Years Later," he stated, "Another important issue is the value of other carotenoids such as lutein.... Would it be more protective if another carotenoid (lutein) were used in place of the beta-carotene? ... The answers to these questions await randomized trials. ... The value of either of these carotenoids [lutein or zeaxanthin] remains unproven.... The value of lutein and zeaxanthin remains uncertain, although one or both of these carotenoids may be better than beta-carotene. Additional research is needed to answer this question ..."

Another example of how experts in this particular field of scientific and medical research regard lutein and zeaxanthin is provided by a major report compiled by a large panel of highly respected experts who specialize in retinal diseases. Those experts were brought together in 1998 by the National Eye Institute (NEI), and they were asked to develop strategic proposals and recommendations that would guide the NEI's funding for eye research over the next five years. The committee reviewed a wide range of options and candidate treatments, and in its report (contained as a entire chapter devoted solely to retinal diseases, in a published book entitled *Vision Research)* the committee specifically identified and named about 60 candidate treatments that the experts thought were deserving of careful scientific study and research funding. Even though that panel of experts identified nearly 60 specific research leads, it never even mentioned lutein or zeaxanthin.

It should also be noted that their omission of lutein and zeaxanthin from even a passing mention could not have been a mere oversight due to a lack of available information. Several of the experts who served on that committee had previously published articles that explicitly discussed lutein and zeaxanthin.

As another example of the uncertainties and doubts that surround lutein and zeaxanthin among experts who actually treat eye diseases, the Inventor herein has personal knowledge of a patient who has the "wet" (or "exudative") form of macular degeneration. This disease is characterized by aggressive growth of capillaries in and around certain layers of the retina, and it leads to rapid and devastating loss of vision. The best known treatment for wet AMD is called photodynamic therapy; it uses a drug called verteporfin, which is activated by a laser that is shone directly into the eyes of patients who have taken the drug.

In October 2003, a patient suffering from wet macular degeneration, who was taking zeaxanthin capsules on a daily basis, was scheduled to have a photodynamic treatment using verteporfin, at the Wilmer Eye Institute in Baltimore, which is affiliated with the Johns Hopkins School of Medicine. This patient told his ophthalmologist, one of the top experts in the world on treating wet macular degeneration, that he (the patient) was taking zeaxanthin. The ophthalmologist advised the patient to stop, since (in the opinion of the ophthalmologist) it probably would not help, and it might interfere with the verteporfin/laser treatment. Despite that suggestion, the patient continued taking zeaxanthin, up through the date of the treatment and continuing thereafter.

The results of that treatment, which have been monitored for more than a year as this is being written, have been outstanding, and have been much better than was expected by the ophthalmologist. That discovery is the subject of a recently-filed patent application, and the case summarized above has been supported by additional consistent anecdotal evidence, obtained through a survey of patients who have been purchasing zeaxanthin from ZeaVision LLC (the assignee company herein) and who have also received photodynamic therapy. For now, two points are worth noting:
(i) when advised that a patient suffering from wet macular degeneration was taking zeaxanthin, one of the top eye experts in the world advised the patient that he should stop taking it; and,
(ii) when that discovery was disclosed, in confidence, to the two companies that jointly sell verteporfin, both companies declined and refused to pursue, support, or fund even small-scale pilot tests to determine whether zeaxanthin will actually help improve the outcomes of photodynamic treatments using their drug.

Accordingly, any analyses of zeaxanthin's role in a nutritional supplement for macular protection must take into account the facts and factors described above, which can be briefly summarized as follows:
(i) various recent reports by experts, published in respected and refereed journals, have directly stated that there is no solid and reliable evidence that zeaxanthin actually can or will help protect the retina;
(ii) various published reports explicitly advise physicians who treat patients suffering from eye diseases that it is premature, and ill-advised, for any physician to instruct patients to begin taking any unproven and potentially dangerous supplements;
(iii) when a large panel of world-class retinal experts was asked, in 1998, by the National Eye Institute, to list the best and most promising candidate agents for future research to help prevent or treat retinal diseases, that entire panel, in its collective wisdom and expertise, completely omitted both zeaxanthin and lutein as candidates that should be considered for research, even though the members of that panel were aware of both zeaxanthin and lutein and had even published articles on them, prior to 1998;
(iv) in October 2003, when one of the world's top experts in treating macular degeneration was informed that one of his patients was taking zeaxanthin, the physician specifically advised the patient to stop taking zeaxanthin, since it might interfere with a different treatment that the physician was planning to give the patient; and,
(v) the National Eye Institute has also declined and refused to take any steps that would help advance or support trials of zeaxanthin. Despite specific and repeated requests from the Inventor herein and certain others, the NEI managers in charge of planning the second major AREDS trial (referred to herein as AREDS-2) have effectively refused to support any testing of zeaxanthin in any way that would enable it to be compared against lutein. Instead, since every commercially available supply of zeaxanthin (which is extracted from marigold flowers) contains a very small quantity of zeaxanthin (zeaxanthin content typically comprises only about 1 to about 3% of the lutein content), the NEI is making plans to test a lutein preparation containing a small quantity of zeaxanthin, and the design of the trial will prevent anyone from being able to identify and evaluate how much benefit (if any) was contributed by each of those two agents. In about December 2003, a responsible official at the National Eye Institute informed the Inventor that the NEI intended to add no extra zeaxanthin to the lutein extract (from marigold) that would be included and tested in the AREDS-2 trial. That position within the NEI was made clear from the name of the group that was helping plan the AREDS-2 trial, called the"Lutein/DHA Advisory Group" (the reference to DHA refers to the omega-3 fatty acid that will also be tested in a separate treatment arm). In May 2004, at a meeting of the"Lutein/DHA Advisory Group", the responsible official revised that position and agreed to add supplemental zeaxanthin to the formula, but only to a level of 2 mg/day, while lutein levels remained at 10 mg/day.

That decision was made largely in response to comments, requests, and rationales provided by the Inventor herein. It is described in the official minutes of the May 17 meeting, which were distributed to all attendees at that meeting.

These factors offer powerful evidence that the invention disclosed herein, which rests upon zeaxanthin as the crucial and essential ingredient in multi-component formulations for preventing or treating eye diseases, is not obvious to those who are truly skilled in the art, and who in fact have devoted their careers to trying to prevent and treat eye diseases.

### PATENTS ON NUTRITIONAL SUPPLEMENTS

Topical compositions comprising lutein and zeaxanthin for treatment of the aging eye are known from US 6,573,299 B1.

Patent Document US 6,649,195 B1 discloses a nutrient formulation comprising 880 mcg of zeaxanthin.

US 2001/031744 A1 discloses nutrient and therapeutic compositions for treating and preventing diabetic retinopathy, macular degeneration and cataracts, the compositions comprising a vast amount of possible components in which zeaxanthin is mentioned, however, without specifically disclosing a formulation comprising 3R,3'R-zeaxanthin.

In US 2001/0009926 A1, compositions comprising meso-zeaxanthin and lutein and their use to increase the deposition of macular pigment in the human eye based upon conversion of lutein in the retina are disclosed.

To complete an analysis of the prior art in this field, it must be noted that, just as numerous nutritional supplement mixtures with various components have reached the marketplace by claiming to offer eye and vision benefits, numerous patents also have been issued on combinations of potentially useful nutrients. Most such patents are largely unsupported by any data concerning actual benefits, and it appears that none of those patents have recognized or addressed the differences between zeaxanthin versus lutein, or the benefits of zeaxanthin over lutein. Instead, nutritional patents that mention zeaxanthin or lutein at all simply mention those two carotenoids in passing, and collectively, apparently because: (i) they are known to be dietary carotenoids, and carotenoids are known to be generally nutritious and healthy, as anti-oxidants, and/or (ii) lutein and zeaxanthin have been reported to be the two macular pigments, and are therefore assumed to be helpful and useful, somehow, in the eyes.

It is impossible to create a complete and reliable list of patents in the field of eye-care nutrition, because most patents on nutritional supplements claim to provide health benefits in various areas other than eye care (such as for skin protection, cancer prevention, cardiovascular health, anti-aging, memory and mental functions, etc.), and because most such patents attempt to claim mixtures of nutrients regardless of their intended use (such patents may discuss eye benefits; they may allude to eye benefits in indirect but suggestive language; or, they may omit any mention of eye benefits, while referring to nutrients that are of interest herein). Nevertheless, the examples listed below can provide the reader with a sense of what has been claimed and asserted in this field.

None of the products that are described or claimed in any of the patents below have reached a level of recognition, use, or sales that equals or even approaches the sales and use of OCUVITE PRESERVISION (with the AREDS formula) from Bausch & Lomb. After publication of the results of the AREDS trial, in October 2001, that product has claimed to be the only nutritional product that has ever been "clinically proven" to help treat macular degeneration, and it now dominates the field of nutritional products for eye health (at least in the United States). Therefore, the patents listed below, and the products they cover (if such products even exist), have only small and limited recognition and use, and none of them have had serious and substantial impacts on the marketplace, or on medical efforts to prevent or treat macular degeneration or cataracts. The only questionable exception known to the Inventor is a product sold by Sigma Tau under the trademark PHOTOTROP, which contains a mixture of carnitine, DHA, and Co-Q10. It is being sold in Europe, and Sigma Tau is attempting to introduce it into the US market, as this is being written.

With that as preface, the patents listed below fall into the category of patents that claim (or that may claim, or suggest) eye and vision benefits from various combinations of potentially useful nutrients.

US patents 5,075,116 (LaHaye et al 1991) and 5,156,852 (LaHaye et al 1992), both licensed to Alcon, claim methods for treating macular degeneration by a combination of: (i) a plurality of antioxidants, such as Vitamins C and E; (ii) a plurality of cofactors (such as zinc, copper, selenium, and manganese) that will activate metalloenzymes that will help scavenge and reduce oxidants; and, (iii) at least one "glutathione-elevating compound" such as L-cysteine, pyridoxine, and riboflavin.

US patent 5,753,703 (Cavazza et al 1998), assigned to Sigma Tau, claims combinations of carnitine (a sulfur-containing amino acid that helps transport fatty acids into mitochondria, as discussed below) and an omega-3 fatty acid such as DHA (docosahexaenoic acid, also discussed below). Optional agents can be included, such as "vitamins, mineral salts, antioxidizing agents and vegetal fibers". US Patent 6,365,622 (Cavazza et al 2002), also assigned to Sigma Tau, claims combinations of carnitine and lipoic acid, but it is not clear how that patent avoids the Ames patent immediately below.

US patent 5,916,912 (Ames et al 1999) claims "dietary compositions for enhancing metabolism and alleviating oxidative stress", which contain carnitine and a "mitochondrially active antioxidant" that contains a thiol group, such as glutathione, N-acetyl cysteine, or lipoic acid.

US patent 5,955,102 (Gorenbein et al 1999), assigned to Amway, claims softgel capsules containing DHA (the omega-3 fatty acid), lutein, and an "anthocyanoside" (this generally includes darkly-colored plant extracts, such as bilberry extract). This patent offers an example of a patent that completely fails to recognize the benefits of zeaxanthin over lutein; various claims in the patent indicate that lutein should be present in a concentration of "about 1 to 6 milligrams", while zeaxanthin only needs to be present at a concentration of about 0.1 to 0.25 milligrams. Vitamins C, E, or A can also be included.

US patent 6,417,233 (Sears et al 2002), assigned to Sigma Tau, claims mixtures of Co-Q10 (a coenzyme also known as ubiquinone, which helps support and stabilize mitochondrial functioning) and an omega-3 fatty acid (such as DHA). The mixture can also contain "vitamins, mineral salts, antioxidizing agents, amino acids, polysaccharides and vegetable fibers".

US patent 6,515,020 (Cavazza 2003), assigned to Sigma Tau, claims methods for treating memory and mental functions by administering combinations of carnitine and certain types of "stilbene" compounds (such as resveratrol) that can be extracted from various types of plants.

US patent 6,572,899 (Gorsek 2003) offers an example of a patent that may or may not relate to retinal protection. It claims "healthy memory and neuro protection compositions", and it is not clear whether its form of "neuro protection" is intended to include protection of retinal neurons. The composition claims mixtures of carnitine, ginkgo biloba, lipoic acid, blueberry extract, spinach extract, Co-Q10, phosphatidylserine, and phosphatidylcholine.

US patent 6,733,797 (Summers 2004) also provides an example of a borderline patent that may or may not relate to eyes and vision. It claims, "A health supplement composition for mammals for improving memory and cognitive abilities", and it is not clear whether vision should or should not be included within phrases such as "cognitive abilities" or "cognitive improvement", as used in that patent. Regardless, its claimed compositions include a phosphoester (such as phosphatidylcholine, phosphatidylserine, etc.) and a "herbal antioxidant" mixture that apparently must include each and all of barberry, bilberry proanthocyanidins, lemon bioflavonoids, lime bioflavonoids, orange bioflavonoids, curcuma, garlic bioflavonoid, ginkgo biloba, ginseng, gotu kola, grape seed proanthocyanidins, red apple quercetin, red onion quercetin, and Siberian ginseng. The mixture may also contain vitamins, minerals, and amino acids.

As stated at the beginning of this subsection, the list above contains examples of US patents on nutritional mixtures. This list is not exhaustive or comprehensive, and instead is intended to provide the reader with a sense of what has been published. As mentioned above, none of the mixtures listed above has had serious and substantial impacts on preventing or treating macular degeneration, with the possible exception of the PHOTOTROP™ product sold by Sigma Tau, which contains carnitine, DHA, and Co-Q10.

As the final item of background information, certain terms need to be defined and clarified. "Ocular" relates to the eye, and terms such as "ocular-active" can be used interchangeably with other terms such as ophthalmic, eye-related, vision-related, etc.

"Nutrients" as used herein refers to compounds that are found in a normal human diet and/or a healthy human body (including precursors and metabolites of nutrients that are actually in the diet), regardless of whether they are synthesized chemically, or extracted from natural sources. This term is intended to be distinct from drugs, pharmaceuticals, antibiotics, and other "xenobiotic" compounds that are not normally found in natural sources. "Nutritional supplements" as used herein is a subset of nutrients, and includes any naturally-occurring nutrients that have been either: (i) manufactured or formulated in a manner other than in a food source, such as in a tablet, capsule, syrup, powder, etc., or (ii) prepared and/or packaged as a food additive (either within a food product, such as in vitamin-fortified meat, dairy, or baked goods or beverages, or in "food-mixable" forms such as powders that are designed to be mixed with beverages). "Dietary supplement" is used interchangeably with "nutritional supplement" or "nutrient supplement".

Accordingly, one object of this invention is to disclose multi-component orally-ingestible nutritional supplements for protecting eye health in mammals (including humans), which contain zeaxanthin as an essential and critical ingredient, and which also contain at least two or more other agents that act in a synergistic and potentiating manner with zeaxanthin, to provide improved efficacy in preventing or treating eye diseases.

Another object of this invention is to disclose a focused method of approach that will be able to clearly identify ocular-active nutrients that, when added to zeaxanthin, can increase the efficacy of zeaxanthin in preventing or treating eye diseases.

Another object of this invention is to disclose a method (which has intertwined aspects of both scientific research, and a method of doing business) that will sort through multiple competing and confusing products accompanied by unsupported and unreliable advertising and marketing claims, and which will provide (i) elderly people who are suffering from vision loss; (ii) their families, caregivers, and insurance companies; and, (iii) government and charitable institutions that will be forced to bear the brunt of the costs of caring for millions of elderly people who are at severe risk of becoming functionally blind, with genuinely useful and reliable products and information that will be truly effective in preventing an epidemic of blindness, which otherwise will occur as the population ages, and as the long-terms effects of unhealthy high-fat diets gradually take their toll on aging populations in numerous nations around the world.

These and other objects of the invention will become more apparent, through the following summary, description, and claims.

### SUMMARY OF THE INVENTION

Orally ingestible formulations provided in the form selected from the group consisting of capsules, tablets, liquids, and powders for oral ingestion for promoting eye health, and in particular for preventing or treating macular degeneration, are disclosed, containing: (i) 3R,3'R- zeaxanthin, a carotenoid, as a crucial and essential ingredient, in a daily dosage of at least about 3 milligrams; (ii) a dosage of elemental zinc not exceeding about 40 mg/day, and preferably in a range of about 10 to about 30 mg/day; (iii) ascorbic acid (vitamin C); (iv) at least one tocopherol (such as vitamin E); and (v) at least two or more additional ocular-active nutrients that provide synergistic eye and vision benefits when combined with zeaxanthin and zinc, selected from the following group:
(1) Lipoic acid, preferably in the form of a purified or enriched naturally occurring "R" (dextrorotatory) stereoisomer rather than a racemic mixture.
(2) At least one omega-3 fatty acid, such as docoso-hexaenoic acid (DHA) or one of its linolenic acid precursors, preferably obtained from a natural source such as fish oil or marine algae.
(3) Various plant-derived compounds that can be referred to as terms such as flavonoids, bioflavonoids, anthocyanins, plant polyphenolics, or phytonutrients. These compounds can include non-specific extracts from various plants (such as bilberry, grapeseed, or green tea), and/or they may contain known and specific molecular components, such as genestein, quercetin, diazedem, fisetin, luteolin, resveretrol, and pycogenol.
(4) Taurine, the common name for amino-ethane-sulfonic acid, a"conditionally essential nutrient"that is present in milk and various tissue types.
(5) Carnitine, a sulfur-containing amino acid (not one of the 20 primary amino acids used in protein synthesis) that is formed in the liver and elsewhere, and various esters and/or precursors of carnitine, such as acetyl-L-carnitine.
(6) An enzyme cofactor known as Coenzyme-Q10, which is a known anti-oxidant that provides energy-related support to mitochondria, and that may help prevent or reduce a process called"apoptosis"that leads to cell death under conditions of stress or senescence.
(7) Carnosine, a di-peptide formed from alanine and histidine, which can prevent reactive aldehydes from causing unwanted glycosylation or crosslinking of proteins.
(8) Nutrients that can stimulate the production or metabolism of glutathione, a tripeptide that helps cells eliminate waste products. One such agent is N-acetyl cysteine, an ester that is metabolized to release the cysteine, the sulfur-containing amino acid in the center of glutathione.

Specific formulations are disclosed and claimed herein, which have different dosage levels, for either of two types of use. Relatively high-dosage formulations are disclosed, for treating people (or other higher mammals) who are suffering from a known and ongoing ocular problem; and, lower-dosage formulations are disclosed, for preventing problems and for preserving eye health and vision in people who are not currently suffering from an ongoing ocular problem.

In addition, an ongoing testing program is disclosed herein, to evaluate and rank the synergistic efficacies of the candidate ocular-active nutrients listed above, both with and without zeaxanthin and zinc coadministration. This testing program will comprise both: (i) selected tests that utilize a balanced combination of several different animal models, each of which can reveal information about certain specific aspects of eye health and eye disorders;
and, (ii) clinical trials on humans, including "meta-trials" that will allow numerous participating optometrists and ophthalmologists to work with a relatively small number of patients, by gathering data on such patients and then submitting the data to a single evaluation expert or group that will carry out consistent and controlled analysis of photographs and other data that are submitted. In addition, means are disclosed for accelerated the steps of data gathering and analysis, such as by focusing data-gathering efforts on selected patients or animals that are entering certain known stages of a degenerative process that involve a period of accelerated and rapid degeneration, such as a stage of dry macular degeneration called "geographic atrophy". Each type of animal model or human clinical trial can provide certain types of data, and will relate to certain specific components of the eye, and/or certain ocular disorders. Accordingly, a testing regimen that involves a balanced selection of specific tests that use targeted yet balanced data-gathering models and methods will be used, rather than unconnected and uncoordinated approaches that generate "scatter-shot" data that are difficult to evaluate and apply to specific cases.

This coordinated testing approach, using zeaxanthin as an essential agent, combined with zinc at a dosage that is safer for elderly people than is contained in the current AREDS products, must be compared and contrasted with the current state of the art. The uncontrolled and unsupported profusion of nutritional supplements, each with its own competing, confusing, and often unsupported advertising claims designed to sell products as quickly as possible (rather than support research for the future), is not working adequately, and will not work adequately in the future, unless something happens to alter the landscape in a substantial and useful manner. Patients cannot be sure what to take, physicians cannot be sure what to recommend, and the largest and most powerful companies that sell eye care nutrients have shown, by their actions, that they apparently are determined to either ignore zeaxanthin completely, or relegate it to a minimal and peripheral role in their products, rather than recognizing its crucial role at the center of the macula, and as the single most important ingredient in any nutrient formula that will be truly effective and useful in protecting eye health and good vision.

Accordingly, the current state of the art does not offer any realistic hope of preventing dozens or even hundreds of millions of cases of avoidable blindness, which will occur around the world over the next 20 years unless a new and different approach is created and enabled which is substantially better than the approach that has been used to date by the largest companies that sell eye care products, and by government agencies such as tne national Eye institute in the United States. Accordingly, the testing approach disclosed herein can be regarded as a process that will identify improved synergistic compositions, and those improved synergistic compositions may offer marginal improvements over the formulations that have already been specifically identified and disclosed in the Examples, below. Therefore, such improved synergistic compositions that extend beyond the specific formulations disclosed herein can be regarded as product-by-process compositions.

### BRIEF DESCRIPTION OF THE DRAWINGS

FIGURE 1 (which is prior art) depicts the chemical structures of zeaxanthin and lutein (with an arrow pointing out the misplaced non-conjugated double bond in one end ring of lutein), and beta-carotene (a similar carotenoid that does not contain any oxygen molecules or hydroxy groups).
FIGURE 2 (which is prior art) comprises Figures 2A, 2B, and 2C. FIG. 2A depicts the bilayer structure of an animal cell membrane, formed by two rows of phospho-lipids having water-soluble phosphate "heads", and oil-soluble lipid "tails". FIG. 2A depicts the way a molecule of beta-carotene (which has no oxygen atoms or hydroxy groups) will aligned itself entirely within the oily interior of a cell membrane. FIG. 2B depicts how molecules of zeaxanthin and lutein will align themselves to "span" or "straddle" a cell membrane, in a way that causes their end rings and hydroxy groups to protrude and extend out, beyond the cell membrane's outer and inner surfaces.

### DETAILED DESCRIPTION

As briefly summarized above, this invention relates to ocular (eye-related) nutrients that can act in a synergistic and potentiating manner with zeaxanthin, to protect and/or restore eye health and good vision to a degree that rises significantly above the levels of benefit that can be provided by zeaxanthin alone. For reasons described herein, zeaxanthin is regarded as a crucial and essential ingredient in such formulations, and it should be ingested at a dosage of at least 3 mg/day, and preferably at least 5 mg/day to sustain general eye health, and preferably at least 10 mg/day to treat a known eye disorder.

A number of candidate additional nutrients are also listed below, and steps and methods are described for determining which of those agents will work in a synergistic and potentiating manner, when ingested with zeaxanthin, to provide significantly better benefits than can be provided by zeaxanthin alone.

It must be noted that various published articles and patents have already suggested that zeaxanthin may be able to provide some benefits for eye health, and for preventing or treating macular degeneration. However, as noted in the Background section, there is ample evidence which clearly shows and proves that even though a few items have been published indicating that zeaxanthin may help prevent or treat macular degeneration, it has not been accepted as such by the large majority of experts who actually work in this field of research or medicine. As summarized in the Background section, such evidence includes, for example:
(i) published articles by experts, saying that zeaxanthin should not be prescribed or even recommended by physicians, until it has been proven both safe and effective by means of intervention trials;
(ii) the decision by a large committee of retinal experts to not even mention zeaxanthin (or lutein), not even in passing and even though about 60 other candidate research leads were explicitly mentioned by name, in a major effort (sponsored by the National Eye Institute) to identify promising research leads that the experts believed deserved careful study and research funding.
(iii) the decision in May 2004, by the managers of the AREDS-2 trial being organized by the National Eye Institute, to run that trial as a "Lutein/DHA" trial, and to test a high dosage of lutein but only a low dosage of zeaxanthin (arising mainly from the fact that zeaxanthin is an unavoidable byproduct of commercial lutein production extracted from marigold flowers), in ways that will not allow the actual benefits of either the lutein or the zeaxanthin to be measured and evaluated; and,
(iv) the position taken by a world-class expert in ophthalmology and macular degeneration, who explicitly and directly advised a patient to stop taking zeaxanthin when he (the ophthalmologist) learned that the patient was taking it, because (in the opinion of the ophthalmologist) the zeaxanthin likely would not do any good, and it might interfere with photodynamic therapy (using a laser beam to activate verteporfin in retinal capillaries) that was being planned for the patient.

In view of and in response to those prevailing attitudes, the following narrative compiles a number of factors that (i) explain more about the roles of carotenoids in general, in the eyes, and (ii) explain and describe certain differences between zeaxanthin and lutein. While most of these factors can be gleaned, piece by piece, from various previously unrelated sources, these factors have not previously been compiled and correlated in this manner, and they apparently are not adequately recognized, understood, or appreciated by those skilled in vision research or ophthalmology (for example, most experts in medicine, vision research, and ophthalmology likely have never studied photosynthesis in detail, and probably are not aware of why lutein has major benefits over zeaxanthin, in "light-harvesting structures" involved in photosynthesis, in plants).

Accordingly, the following sections are provided as a compiled teaching, containing information that has not previously been recognized and correlated be experts in medicine and ophthalmology, in ways that support and comprise a significant part of this invention.

### CAROTENOID BENEFITS BEYOND PROTECTION FROM UV AND RADICALS

One of the contributing factors that led the Inventor to recognize that a carotenoid such as zeaxanthin is both (i) a crucial ingredient that is essential for eye health, and (ii) an "anchor" ingredient that will enable other useful agents to work more effectively and in a synergistic manner, was the gradual realization, which arose over a span of more than a decade of reading thousands of articles, abstracts, and patents on carotenoids, of how many different roles carotenoids can play. In particular, carotenoids can play any or all of several different roles that extend above and beyond their well-known roles in absorbing ultraviolet radiation and quenching oxygen radicals.

Since most medical researchers and ophthalmologists apparently are not aware of the activities and factors listed below, or have not yet recognized how these numerous contributing activities and factors cumulatively enable zeaxanthin to provide a remarkable range of benefits to people suffering from eye problems, a numbered list is provided, directly below, which briefly touches on half a dozen of the lesser-known activities of carotenoids.

One of the factors that has caused these activities to be overlooked and ignored, by medical researchers, is that all of these activities can generally be described as offering only mild, weak, and partial levels of benefit. Therefore, when it comes to matters such as designing, organizing, and paying for clinical trials to prove that these benefits can be important, these potential contributing factors fall into a highly doubtful and unreliable zone, where they do not receive serious attention. This factor is aggravated by certain types of biases that are built into clinical trials, including factors that center around what is called the "standard of care" for any particular type of disease or disorder that is being tested. Briefly, the "standard of care" issue, which arises in designing and conducting clinical trials, generally means that it is unethical, and often even illegal in ways that can lead to lawsuits and huge damage awards, for a company to withhold from patients a treatment that is known to be effective in treating a certain type of disorder that such patients may be suffering from.

As an illustration of this doctrine, it would be effectively impossible to carry out a human clinical trial to prove that carotenoids have a relatively low yet beneficial level of activity in helping prevent or control ocular inflammation in humans, because there are other known treatments (mainly anti-inflammatory steroids) that are much more targeted and potent in treating that particular problem. To properly test carotenoids for anti-inflammatory effects in humans, the best known treatments would need to be withheld from all patients being tested, including "control" patients who would receive nothing but ineffective placebos to establish comparative data from untreated subjects. However, in tests on humans suffering from a serious problem that has a good and effective treatment, withholding known effective treatments even from "control" patients would be unethical, and improper. This effectively makes it impossible to carry out a clinical trial to prove that some carotenoid can provide a mild but potentially useful benefit against a serious problem (such as treating ocular inflammation, to continue the example offered above), when other agents are already known to be more effective in treating that problem.

For those and other reasons, physicians, scientists, and other experts regard the factors listed below as being unproven and unreliable, without sufficient support to extrapolate any data from cell culture or animal tests to actual human medicine. Therefore, in the consensus view of most physicians, scientists, and other experts, the beliefs set forth below, no matter how sincere they may be, are not adequate to support medical recommendations by physicians who must diagnose and treat patients suffering from macular degeneration or other eye or vision problems.

The Inventor herein is acutely aware of the difficulties in proving that zeaxanthin can provide the types of useful but relatively mild and weak benefits described below. However, he has recognized how these types of relatively mild and weak benefits can be woven and incorporated into a larger package of active agents that, together, can provide significantly stronger benefits that will be able to overcome and surpass the hurdles and requirements that apply to human clinical trials.

In addition, it should be noted that various articles describing apparently unconnected aspects of carotenoids gradually accumulated, in the overall understanding and perspective of the Inventor, until they led to an insight and recognition by the Inventor that has never been suggested or addressed in any prior art. Accordingly, the information below, on a number of relatively weak activities of carotenoids, is regarded as part of this invention, and the Inventor suggests and teaches herein that these factors, taken together, must be combined and connected into larger cohesive framework that merits serious and careful attention by physicians, ophthalmologists, and optometrists.

Accordingly, the following factors, all of which led to a specific insight that supports and substantially contributes to this invention, need to be recognized and given due consideration:
(1) Carotenoids have a mild ability to help control and reduce inflammation, as described in articles such as Ohgami et al 2003, Lee et al 2003, Gonzalez et al 2003, Ford et al 2003, and van Herpen-Broekmans et al 2004. Although their effects in this area are not as potent as anti-inflammatory steroids, these effects may nevertheless become important, in significant numbers of patients suffering from eye disorders, because any inflammation that involves or affects either or both eyes is an important threat and risk factor, and can lead to serious and even severe adverse results, including blindness.
   Even a relatively slight episode of inflammation, if it directly affects either or both eyes, can permanently damage the eyesight, if the inflammation leads increased fluid pressure involving the vitreous humor (i.e., the jelly-like clear liquid between the lens and the retina). Except in the small macular region at the center of the retina, the capillaries that serve the retina actually sit on the front surface of the retina, directly exposed to fluid pressures, rather than being embedded within a structural layer. Therefore, if fluid pressures increase in the vitreous humor, those elevated fluid pressures will press directly against the exterior surfaces of the retinal capillaries. Since capillary walls must be extremely thin, to promote rapid exchange of oxygen, nutrients, and metabolites, they cannot resist and push back, if elevated fluid pressures are imposed on them. Therefore, following basic principles of fluid flow, even a slight elevation in the fluid pressure of the vitreous humor, in an eye, can cause a significant portion of the blood that normally flows through retinal capillaries to be diverted. The blood supply that the retina needs will simply take a different route, somewhere else in the body, at any other location where the capillaries are not being squeezed and compressed.
   This mechanism explains why glaucoma will cause blindness if not treated.
   Glaucoma is actually the name given to an entire class of eye diseases that share a common trait: they involve elevated fluid pressures inside the eye. This pressure elevation can be caused by any of several factors (such as the secretion of too much fluid by certain types of eye tissues, factors that hinder drainage and flow through the drainage ducts, etc.). Regardless of the cause, any disorder that involves chronic elevated pressure inside the eye is called glaucoma, and it will lead to blindness, since elevated pressures will hinder the flow of blood through the retinal capillaries.
   If elevated fluid pressures inside the eye are caused by inflammation due to an injury or infection, the pressure increase may not be chronic or permanent, but it may last for days or weeks, which is more than long enough to inflict severe and permanent damage to the retina. Therefore, the ability of carotenoids to help reduce and control inflammation, even if that beneficial activity is only mild and weak when compared to potent drugs such as steroids, may be extremely helpful and crucially important in protecting eyes and vision against permanent damage caused by decreased retinal blood flow, caused by inflammation due to an injury or infection.
   It must also be recognized that anti-inflammatory steroids cannot be given to patients for long periods of time, without causing serious side effects (as can be observed in patients who must take steroids for extended periods, due to diseases such as lupus). Therefore, even though anti-inflammatory steroids are highly useful for treating acute inflammation following an infection or injury, they are not useful or desirable for most types of long-term use. By contrast, carotenoids can and should be a part of a normal and healthy daily diet for a person's entire lifetime.
(2) Carotenoids also have mild yet potentially helpful ability to prevent and reduce sclerosis, as described in articles such as Carpenter et al 1997. "Sclerosis" refers to hardening, stiffening, and loss of flexibility (for example, arteriosclerosis refers to hardening of the arteries, while atherosclerosis is a related process in which the insides of the arteries become coated with cholesterol or other fatty deposits).
   In the eyes, sclerosis and loss of flexibility can arise not just in blood vessels, but also in certain layers and structures of the eye, especially if substantial quantities of drusen, lipofuscin, and other debris accumulate in those layers and structures. In addition to rendering the eye less able to focus on objects at varying distances, this loss of flexibility can damage certain membranes, such as the Bruch's membrane, a crucially important layer behind the retina. Therefore, by helping prevent and reduce sclerosis, even if only mildly, carotenoids can help protect eye health and good vision.
(3) Carotenoids also have mild yet potentially useful levels of activity in controlling and regulating angiogenesis (i.e., the formation of new blood vessels), because of their ability to help suppress various angiogenic hormones and cytokines, as described in articles such as Armstrong et al 1998 and Chew et al 2003. Since the formation of new blood vessels can lead to severe problems and blindness in "wet" or "exudative" macular degeneration, this activity of carotenoids may offer significant advantages not just in treating wet macular degeneration, but in preventing it in the first place.
(4) As described in articles such as Chew et al 2003, carotenoids have mild yet potentially useful levels of activity in helping to support and stabilize mitochondria, which are "organelles" inside cells. Typical cells contain anywhere from a few dozen to several hundred mitochondria, which have two major functions. First, they control and carry out processes that involve oxygen usage, respiration, and energy usage. Second, they control a process called "apoptosis" (sometimes called programmed cell death), which triggers a process of accelerated death, digestion, and recycling, when an aging or injured cell declines to a non-viable state. This process helps enable the gradual turnover and replacement of cells in animal tissues. It is governed by mitochondria, which carry their own genes and use a genetic code that is different from the genetic code used by chromosomes in the nucleus of a cell (mitochondria actually are the descendants of tiny bacteria that invaded larger cells, soon after the beginning of life on earth, and then set up a symbiotic relationship with their host cells).
   Apoptosis is a well-known process, involving a series of messenger molecules released by the mitochondria when the cell enters the last phase of its life. Briefly, under various conditions that indicate senescence or in response to certain "upstream" signals, the membranes of mitochondria inside a cell become porous and leaky, and they begin releasing a messenger called "cytochrome c". The release of cytochrome c triggers downstream signals that cause killer cells with long fibrous extensions (called "dendrites") to move in, finish the job quickly, and digest the dead cell, so its "building blocks" (amino acids, nucleotides, lipids, etc.) can be recycled and used to create a new and healthy replacement cell.
   Apoptosis is crucial and necessary, in nearly all types of animal tissues. However, two major factors need to be recognized about it. First, in adults, neurons in the central nervous system (CNS) are exempt from that process, since CNS neurons cannot be regenerated and replaced. CNS neurons mainly are in the brain and spinal cord, but they also include retinal neurons, since the retina is recognized and classified as a specific type of CNS tissue.
   The second major factor is this: like any other biological process, apoptosis sometimes can go wrong, and it can suffer from various disorders. In apoptosis, which by definition leads to the death of a cell, the main problem is that apoptosis can be triggered and activated prematurely, by various types of stress or damage that may cause cells to die before they should. If that happens among CNS neurons (including retinal neurons), the damage is permanent, because CNS neurons (including retinal neurons) cannot be regenerated and replaced. As noted above, CNS neurons are supposed to be exempt from apoptosis, at least in adults; however, if stress becomes severe, and if the mitochondria in a cell begin to stagger under the strain of a load that is chronically too heavy, apoptosis can be triggered even in retinal or other CNS neurons.
   Accordingly, if carotenoids have even mild benefits in helping stabilize and support mitochondria that are struggling under abnormally heavy loads, they may be able to help stave off apoptotic cell death, in retinal and CNS neurons that cannot be regenerated.
   It should also be recognized that this potential benefit from carotenoids may be rendered even more likely and useful, if stressed mitochondria can also be helped and protected by other mitochondrial boosting agents as well, such as Coenzyme-Q10 or certain types of glutathione boosters. Such agents are good candidates for coadministration with zeaxanthin, as discussed below.
(5) Carotenoids have mild yet potentially useful levels of activity in helping regulate and control certain types of actions and responses of the immune system, as described in articles such as Walrand et al 2004 and Carpenter et al 1997. These activities may be manifested in ways that relate to inflammation, suppression of dendritic killer cells that otherwise might carry out apoptosis too soon, etc.; however, this type of mild and subtle contribution to proper regulation of the immune system can also be manifested in other potentially useful ways, as well. Accordingly, this factor should be noted, and kept in mind.

It must also be noted that the five "secondary" activities of carotenoids, listed above, also act in addition to the two primary activities of carotenoids, which are (1) protection against destructive ultraviolet radiation, and (2) protection against destructive oxygen radicals).

Upon recognizing that carotenoids (a class of highly specific compounds that cannot even be created by animals, and which therefore must be ingested in animal diets) play at least seven different useful activities, in animals, the Inventor began pondering and looking deeper into various underlying factors and activities. The realizations he gradually reached, during a long period of consideration, gradually fit into a larger framework of study and understanding, involving eye and vision disorders. Several factors and insights that can help describe and explain that framework, and that help show how that framework can be put to good use, focus on "connecting rods" that connect different parts of the frame to each other. Four of those "connecting rods" can be summarized as follows:
(i) oxygen radicals play roles in (or are created in increased quantities by) several different classes of problems, which may manifest in different but overlapping ways, such as in tissue inflammation and immune responses; it should also be noted that elevated quantities of oxygen radicals can trigger the production of inflammatory cytokines, as described in articles such as Ohgami et al 2003, Lee et al 2003, and Armstrong et al 1998, and Ford et al 2003; and, elevated quantities of oxygen radicals are also produced by certain types of immune cells, which use those oxygen radicals to help kill and digest microbes, as described in articles such as Walrand et al 2004;
(ii) mitochondria are also deeply and heavily involved in numerous processes that use or manipulate oxygen, and that can generate oxygen radicals;
(iii) cells have only limited numbers of signalling pathways they can use to communicate with each other; and,
(iv) reports have indicated that people suffering from various types of eye problems also suffer from low carotenoid concentrations in their blood (as shown by tests on blood serum), and in their eyes (as shown by low levels of macular pigment, and low concentrations of zeaxanthin in the lenses of people suffering from cataracts);

Accordingly, after realizing that carotenoids may be called upon to perform a number of different secondary activities in addition to two main primary activities, the Inventor gradually reached two important conclusions about carotenoids in human health, and especially in the eyes. Those conclusions can be summarized as follows:
1. If carotenoids are being asked to perform at least seven different known tasks (and possibly even more), all of which can converge and rise to levels of major importance if and when the eyes begin to suffer from serious problems, then carotenoids are more likely than other types of molecules to become "stretched thin", to a point where their concentrations will drop and they will not be able to adequately handle all of the tasks and problems that are being pushed at them;
2. If any of the "secondary demands" that may tend to "siphon off" the desired concentrations of carotenoids in the eyes can be reduced, by means such as administering other nutrients that can provide a balanced regimen that will help address and satisfy those secondary demands, then any newly-arriving carotenoids will be more likely to actually arrive at locations where they can carry out their essential primary roles and provide the most overall benefit.

Accordingly, over a span of time that allowed extended consideration and additional readings on related subjects, this line of logic and analysis began to suggest more and more persuasively that well-balanced eye-care preparations would be able to do the greatest possible good, in protecting or restoring the extraordinarily complex needs of human eyes, if those formulations contain both: (i) zeaxanthin, as the ideal, symmetric, fully-conjugated carotenoid that has been fully optimized (by millions of years of evolution) for interacting in beneficial ways with animal cells and animal cell membranes; and, (ii) one, two, or more additional ocular-active nutrients that can directly address and handle any "secondary demands" that would otherwise tend to "siphon off" part of any zeaxanthin that reaches the eye.

Viewed from another perspective, zeaxanthin can be regarded as a form of "buffer", in a system that is constantly trying to sustain an equilibrium (often called "homeostasis", when biological systems are involved). Like other types of buffer compounds, carotenoids can respond to whatever is added to (or imposed upon) the system, in a way that usually will help the system move back toward its equilibrium (also referred to as the "set-point" of the system). However, if the outer limits of the buffering capacity of a certain acid/alkali buffer has been reached in a certain system, addition of even a slight quantity of additional acid or alkali can cause major swings and upheavals, in a closed system.

In an analogous manner, if the "buffering system" provided by carotenoids, in a human eye, has already been stretched to its limit by a combination of multiple competing demands, all demanding responses at the same time, then the protective "buffering system" can fail, leading to a series, cascade, or mixture of stresses, problems, and damage, all occurring at once and all acting together, in ways that are suggested by phrases such as vicious circle, witch's brew, etc.

Subsequently, as he pondered various approaches to developing and optimizing ways to treat the complicated and intertwined problems that either lead to, or are caused or aggravated by, various types of intractable ocular diseases and disorders (which lead to severe visual loss and either functional or complete blindness, in millions of people, every year, despite the best efforts of thousands of doctors and researchers), the Inventor gradually arrived at a complex intersection, where roughly half a dozen themes converge and meet. Briefly, those themes include the following:
(i) Using nature and evolution as the best examples and the best instructors, many and probably most of the best candidate ocular-active nutrients are likely to be derived from plants;
(ii) In the same way and for the same reasons that occur in plants, and as a result of the various ways that plants and animals coevolved, many or even most of the best candidate nutrients are likely to have strong or even exclusive specificity for certain stereoisomers; and, therefore, racemic mixtures created by non-specific chemical synthesis should be avoided, wherever possible, in favor of the specific stereoisomers that can be found in plants and nutrients;
(iii) Despite the predominance of plant nutrients in the complete catalog of most-promising candidates nutrients, humans evolved most efficiently as omnivores, and diversity should be recognized, respected, and valued. Accordingly, animal sources may well offer a few ocular-active nutrients that can provide good and useful complements, when added to best-candidate plant nutrients for eye health; and,
(iv) after a list has been developed that contains the best candidates from the realm of naturally-occurring ocular-active nutrients, the final step is to make good, shrewd, intelligent use of technology and testing methods, to (i) select the best overall combination of nutrients, and then (ii) get those selected nutrients stored, packaged, and delivered in the most economic and efficient way possible. In this context, appropriate technological steps can include, for example: (i) the use of oily carrier substances, to deliver active agents (including carotenoids) that are naturally oil-soluble; (ii) the use of timed-release and/or sustained-release technology, to establish sustained and lasting increased blood concentrations of any compounds that otherwise disappear rapidly from the gut or from circulating blood; and, (iii) the use of various types of bioavailability enhancers (such as bile salts, phospholipids, or pancreatic lipase), to increase the uptake of oily compounds through the intestinal walls, and into circulating blood.

Once the reader has reached a working knowledge of the benefits of carotenoids in protecting ana improving eye nealth, attention must now turn to explaining why zeaxanthin is the optimal and ideal carotenoid for use in protecting and improving animal and human health, including eye health.

### COMPARISONS BETWEEN ZEAXANTHIN AND LUTEIN

In order to adequately compare, contrast, and understand the different contributions of lutein versus zeaxanthin in eye health, a number of relevant factors must be considered. As with the description above, most of these factors simply are not known to physicians, ophthalmologists, optometrists and other who have become skilled in diagnosing and treating medical and vision problems. Instead, many of the facts listed below are relatively obscure, and are known mainly to botanists and chemists who specialize in plants or carotenoids, rather than in medicine or vision research.
(1) Lutein is bent (or "kinked") at the end that contains the "epsilon" ring. This bend, near one end of the molecule, enables lutein to fit into circular "light-harvesting structures" in chloroplasts, which are organelles in plant cells that play major roles in photosynthesis.
(2) Since zeaxanthin has "beta" rings at both ends, it is straight, with no bend or kink. Therefore, it cannot fit properly into the circular light-harvesting structures that are crucial for photosynthesis, in plants. Even in plants where zeaxanthin does occur, it does not accumulate in substantial quantities; instead, it becomes part of a daytime/nighttime cycle, which shuttles the molecule back and forth between zeaxanthin, and a different carotenoid called violaxanthin.
(3) Because of its role in light harvesting structures, lutein became a heavily dominant carotenoid in plants, while zeaxanthin is present only in very small trace amounts. Even in dark green plants with high concentrations of zeaxanthin (such as spinach or kale), concentrations of lutein are dozens or hundreds of times greater than zeaxanthin.
(4) Although lutein is dominant in plants, which must be able to harvest light and carry out photosynthesis, animals do not perform photosynthesis, and animal cells do not have, use, or need chloroplasts, or light-harvesting structures. Therefore, the advantages of lutein, a bent-chain molecule that fits ideally into circular light-harvesting structures in plants, become totally irrelevant, in animals and animal cells. After ingestion by an animal, the advantages of zeaxanthin, over lutein, move to the forefront.
(5) Zeaxanthin has a higher level of *conjugation* than lutein. As mentioned in the Background section, this term refers to the fact that when bonds between adjacent carbon atoms alternate between single bonds and double bonds, in a regular and repeating manner, the electrons that form those bond take on a "shared" arrangement, and create what is often referred to as an "electron cloud". This same result also occurs in benzene rings and other "aromatic" molecules, discussed in any textbook on organic chemistry.
(6) As can be seen from the molecular structures of zeaxanthin and lutein, in FIG. 1, the "straight chain" portion of both molecules (this "straight-chain" portion is actually a zig-zagging chain, which connects the two end rings) is fully conjugated, and surrounded by an electron cloud. This is conventional for many and even most carotenoids, and the differences between them arise in their end rings.
(7) In zeaxanthin, the conjugated electron cloud covers parts of *both* of its two end rings, since the alternating sequence of single and double bonds extends into both of zeaxanthin's two end rings (which are both "beta" rings).
(8) By contrast, in lutein, one of the rings is an "epsilon" end ring, which does not contain or extend an alternating sequence of single and double bonds. Therefore, the conjugated electron cloud of lutein covers a portion of only one of the end rings of lutein (which is a beta ring); it does not cover any part of the "epsilon" end ring of lutein.
(9) A conjugated electron cloud is absolutely crucial to the two main protective activities that caused carotenoids to become widespread in the plant world. A conjugated electron cloud enables carotenoids to absorb ultraviolet and near-UV radiation, without being badly damaged and broken apart; and, a conjugated electron cloud enables carotenoids to quench and neutralize oxygen radicals, which contain aggressive and unstable unpaired electrons. Carotenoids evolved and became widespread, in plants that are exposed to direct sunlight for hours each day, because their conjugated bonds and electron clouds render them ideally suited to deal with both of those threats to plant cells and tissues. Subsequently, carotenoids from plants were adapted and used by animals to serve the same protective roles, since animals cannot synthesize them and must eat them in their diets.
(10) As mentioned in the Background section, because of the hydroxy groups attached to their end rings, zeaxanthin and lutein are deposited in ways that cause them to "span" or "straddle" the outer membranes of animal cells. This places zeaxanthin or lutein in a direction that is perpendicular to a cell surface, with a portion of each end ring protruding from both the interior and exterior surfaces of the cell membrane.
(11) When zeaxanthin has been deposited into an animal cell membrane, its straight-chain structure is ideal, since it allows zeaxanthin to fully extend portions of both of its two end rings (with their protective electron clouds) slightly beyond both of the inner and outer surfaces of an animal cell membrane.
(12) By contrast, the bent and kinked structure of lutein hinders and impedes its ability to properly span an animal cell membrane. Indeed, some *in vitro* tests using liposomes have suggested that part of the lutein in an animal system doesn't even straddle cell membranes at all, and instead remains within a membrane interior, in a manner similar to beta-carotene.
(13) Even when lutein molecules do straddle an animal cell membrane, they can provide a conjugated and protective (UV-absorbing, radical-quenching) electron cloud on only one side of that membrane, where the "beta" ring is located. As mentioned above, the epsilon ring at the other end of a lutein molecule has no protective conjugated cloud at all.

As a result of these factors (and possibly others), it is believed by the Inventor that zeaxanthin can perform, in humans, in ways that can provide greater benefits than lutein, especially in eye tissues.

This belief is also supported by certain other known factors. First, it is known that the retina deposits zeaxanthin preferentially over lutein. Zeaxanthin is deposited at the highest concentrations directly into the crucial center of the macula, while lutein is deposited at higher concentrations around the edges and periphery of the macula. While the mechanisms that enable this to occur are not fully understood, it recently has been reported that certain enzymes that appear to be involved will clearly bind to zeaxanthin with relatively high affinity, under *in vitro* conditions, but those same enzymes will not bind to lutein with any significant affinity. This is a potentially very important finding, described in Bhosale et al 2004.

Second, it is also known that the macula attempts to convert lutein into zeaxanthin. However, the conversion process cannot convert lutein into the normal stereoisomer of zeaxanthin found in nature (the 3R,3'R stereoisomer); instead, it converts lutein into a different stereoisomer that has never been found in any food sources or mammalian blood. That non-dietary isomer has one end ring with the conventional "R" configuration, but the second end ring has an unnatural "S" configuration that is not found in any dietary sources. That S-R isomer is often called *meso*-zeaxanthin.

Except for very small amounts (measured in nanograms) that can be found in human retinas as a result of the lutein conversion process, *meso*-zeaxanthin has never been found in nature, or in any plant or food sources. This assertion requires an explanation and defense, since it contradicts a claim that was published in 1986 by researchers working in Japan. Maoka et al 1986 asserted that *meso*-zeaxanthin had been found in certain types of marine life, such as in the skins of certain types of fish. However, that claim was directly contradicted, years later, by Khachik et al 2002, which showed that the type of alkaline processing that was used by Maoka et al, actually caused lutein to be converted into *meso-*zeaxanthin, because of a chemical process that apparently was first described in Bone et al 1993. In other words, the alkaline extraction and processing steps that were used by Maoka et al to extract and treat carotenoid samples created *meso*-zeaxanthin as a misleading artifact. It was not actually shown to exist in nature, and instead was created by the alkaline processing used by Maoka et al. This becomes clear from studying column 2 of page 3388 of Khachik et al 2002, which reported that:
(1) When the same processing steps used by Maoka et al were also followed by Khachik et al, *meso*-zeaxanthin appeared to be present in human blood; but,
(2) When more modern and accurate processing methods were used by Khachik et al, *meso*-zeaxanthin was shown to be not present, at all, in human blood.

In other words, Khachik et al 2002 directly showed that the chemical processing used by Maoka et al in the 1980's could and would create *meso*-zeaxanthin, when carried out on a biological fluid sample. Therefore, no one can accurately or reliably claim that *meso*-zeaxanthin has ever been found in any dietary source, and any such claims based on the 1986 article by Maoka et al are misleading, unsupported, and directly contradicted by later (and more accurate) research.

Since *meso*-zeaxanthin has never been found in human blood, there is a consensus that any *meso*-zeaxanthin found in human maculas must be formed by a lutein conversion process. Accordingly, the fact that the human macula does indeed try to convert lutein into zeaxanthin (resulting in a stereoisomer that does not otherwise exist in nature) is strong evidence that the human eye "prefers" zeaxanthin over lutein.

For the reasons summarized above, the Inventor herein believes and directly asserts that zeaxanthin can, does, and will offer substantially better protection than lutein, for human eyes and vision. However, the Inventor will also agree that this belief and assertion should be tested and evaluated, by studying hard data generated by animal tests and clinical trials. Accordingly, a number of specific trials for testing his assertion are described below.

Two additional points should be noted. First, it is believed that treatment with zeaxanthin is believed to be entirely free of any significant risks, among people who are suffering from macular degeneration or other eye problems. That statement is supported by a "New Dietary Ingredient" application that was filed on zeaxanthin, with the U. S. Food and Drug Administration, by Roche Vitamins, Inc. That NDI application contained extensive safety data, including data from animal tests indicating that even at very high dosages, zeaxanthin did not cause any pathological changes, of any sort, in animal tests.

The docket number of that NDI application, published in June 2001, is 95S-0316. It can be downloaded at no charge from the FDA website. Accordingly, it is believed that clinical trials as proposed herein can be carried out without any significant risks to any patients.

Second: this is a very low-cost treatment, compared to the costs of either (i) macular degeneration, which often leads to functional blindness, or (ii) verteporfin-laser (photodynamic) treatments, for the small minority of patients who suffer from the "wet" form of macular degeneration. For example, each verteporfin-laser treatment costs thousands of dollars. By contrast, 60 capsules of zeaxanthin, containing 10 mg each, cost roughly $60.

### ZEAXANTHIN ISOMERS AND ESTERS

Because of its close similarities to the natural dietary 3R, 3'R stereoisomer of zeaxanthin, the non-dietary S, R isomer called meso-zeaxanthin may be able to generate some level of protective benefits, if used in addition to natural and dietary 3R, 3'R-zeaxanthin. Accordingly, any references herein to "zeaxanthin" are specifically intended to include the "meso" isomer of zeaxanthin. However, the claims below only cover the 3R, 3'R stereoisomer form of zeaxanthin.

However, any suppliers or ophthalmologists who might wish to consider selling, using, or prescribing meso-zeaxanthin for human patients should be aware of several concerns and questions that should be evaluated carefully, before any such usage commences. Those questions and concerns include: (i) as summarized above, Khachik et al 2002 contradicts the claim in Maoka et al 1986 that meso-zeaxanthin was found in certain types of marine life, and it has never been found in any natural dietary sources; (ii) if meso-zeaxanthin is indeed a non-dietary isomer, as stated in a number of published articles, and if it is being manufactured by chemical treatment of lutein, then the statutory language in the Dietary Supplement Health and Education Act (the DSHEA statute, passed by Congress in 1994) would appear to require certain legal requirements to be met, before it can be sold or administered to humans. In particular, the language concerning "chemically altered" food compounds, in section 413 of the DSHEA act (codified in 21 U. S. Code 350b) needs to be evaluated carefully, and a recent search of the publicly accessible records on the U. S Food and Drug Administration's database indicates that the requirements of that section have not been met, and none of the legal and regulatory filings have been made that apparently would be necessary to enable lawful sales of meso-zeaxanthin to human consumers, or to any other company that would feed it to poultry or salmon as a pigment, or otherwise put it into the human food chain; (iii) when a test was done in Spain to compare a meso-zeaxanthin preparation against a zeaxanthin preparation containing the dietary 3R, 3'R stereoisomer, as a color additive for poultry, the meso-zeaxanthin preparation did not perform satisfactorily, as described in Perez-Vendrell et al 2001. In addition, based on a website posting sponsored by Texas A & M University describing an annual conference on poultry feeds and pigments, supplemented by inquiries by an industry consultant, it is believed that a similar test was done in Mexico, which also showed unsatisfactory pigmenting results for meso-zeaxanthin in poultry. Those results apparently were never published; however, they raise serious questions about the deposition of meso-zeaxanthin in animal tissues.

Accordingly, the natural and dietary 3R, 3'R isomer of zeaxanthin is strongly preferred over the meso isomer of zeaxanthin.

In addition, while the claims below only concern the 3R, 3'R stereoisomer form of zeaxanthin, any references herein to "zeaxanthin" include esters of zeaxanthin. Most plants and many bacteria synthesize lutein and zeaxanthin, not in the form of "free" carotenoids (also called"alcohol"carotenoids), but with one or two fatty acids linked to the hydroxy groups in a manner that creates an ester bond. However, when these esters are ingested by animals, most of the ester linkages are broken, in a manner that releases free (non-esterified) zeaxanthin or lutein. This chemical reaction is often called "hydrolysis", since a water molecule is effectively inserted into what was previously the ester bond. These reactions are catalyzed by esterase enzymes, and various other digestive enzymes. Accordingly, since zeaxanthin or lutein esters will release free zeaxanthin or lutein after ingestion by an animal, they are regarded as nutritionally equivalent, and any reference herein to any carotenoid that contains a hydroxy group also includes the ester form.

### REDUCED ZINC DOSAGES

As described in the Background section, the AREDS clinical trial that was done in the 1990's tested dosages of 80 mg/day of elemental zinc, and the two main brands of AREDS supplements that are being sold in the US (under the trademarks OCUVITE PRESERVISION by Bausch & Lomb, and I-CAPS AREDS by Alcon Laboratories) both contain 69.6 mg/days dosages of zinc.

However, zinc has been shown to be heavily involved in the formation and growth of B-amyloid plaques, which are a key factor in triggering or aggravating Alzheimer's disease, as described in articles such as Bush et al 1994, Cherny et al 2001, Ritchie et al 2003, Bush 2003, Finefrock et al 2003, and Cuajungco et al 2003. Zinc also has been shown to increase the risk of aggravated brain damage during and after a crisis that deprives the brain of blood or oxygen, such as a stroke, cardiac arrest, or head injury, as described in articles such as Choi et al 1998, Lee et al 2002, Sheline et al 2003, Canzoniero et al 2003, Ryu et al 2002, Manzerra et al 2001, Lobner et al 2000, Suh et al 2000. None of those reports were ever cited or addressed in any of the first 11 AREDS reports that were published, and AREDS Report #12 (Yaffe et al 2004) cited only the Bush 1994 paper and raised more questions than it answered, as summarized in the Background section, above. In addition, articles such as Linkous et al 2004 have reported that in laboratory animals, dietary intake of excess zinc leads to elevated zinc levels in brain tissue and to impaired mental performance in those animals, and articles such as Leitzmann et al 2003 have described an apparent correlation between heavy zinc intake, and prostate cancer.

In the US, the Institute of Medicine issued a formal set of guidelines in 2001, declaring that the "Tolerable Upper Intake Level" for any adult is 40 mg/day. However, it appears that neither Bausch & Lomb nor Alcon Laboratories have recognized or addressed that fact in any labeling or advertising for their AREDS products; instead, both of those companies continue to make labeling and advertising claims for their products based on "Recommended Daily Allowance" numbers that are old, outdated, and no longer correct.

Accordingly, serious questions arise as to whether the extra-heavy zinc dosages that are present in the products currently being sold with AREDS formulations are safe, or dangerous, especially when ingested every day by people in their 70's and 80's, who have elevated risks of amyloid plaque formation and Alzheimer's disease, and of strokes, cardiac arrest, and other excitotoxic crises.

Regrettably, the AREDS-1 trial tested only a single dosage of zinc, and did not study any additional dosages that could have helped generate a curve that would provide more insight into how different dosages of zinc would affect macular degeneration.

One of the challenges, in establishing optimal dosages of zinc that can and should be ingested by millions of people over the age of 60, is that those consumers do not all fit cleanly and neatly into a single, simple category. Instead, they will be distributed across statistical "bell curves" in a number of important respects (including age, body weight, overall medical condition, eye health, other medicines being taken, risk or history of Alzheimer's disease, and risk or history of stroke or cardiac arrest).

However, a highly useful guiding principle can be safely relied upon, with respect to any and all such consumers. That principle has been expressed and encapsulated in a phrase developed by economists, which is *diminishing marginal utility.*

The word "marginal" in this context is used in the same way as in "marginal tax rates", which focuses not on the total taxes a person or company must pay, but on what will happen to the *next* dollar that is earned, if a person's or company's income increases. A "marginal" analysis does not focus on what happened in the past, or on numbers calculated as averages. Instead, it accepts whatever happened in the past as a fixed and unchangeable fact, and then it focuses on the question of what happens next, on a "going forward" basis. Instead of asking, "How much total good has this particular material provided to us?", a marginal analysis asks questions such as, "Since we already have X amount of this material, how much more do we really need, and how much should we pay to get more? What will we do with the next batch that arrives?"

The reference to *diminishing* marginal utility reflects the fact that if a system is dynamic, adaptable, and somehow responsive and/or "intelligent", then the first delivered quantity of some new and useful material can be put to very good use, to solve critical or even dire needs. However, after the most dire and pressing needs of a system have been met by making good use of a newly-delivered material, additional loads of the same material, delivered to the same location, organism, or system, will not provide as many benefits as the earlier batch(es).

Since this invention relates to nutrition, a simple example can be provided by food. If a man is hungry, the first plateful of food he gets will be greatly appreciated. The next plateful may still be good, but it won't be as good as the first. If he then eats a third plateful of food, it won't be because he's still hungry; and then, if he eats a fourth plateful of food, after already finishing three full plates, it's going to do him more harm than good.

As another simple example, every farm needs rain; it needs water, to enable crops to grow. However, too much rain leads to flooding, which can be every bit as destructive as drought.

The examples above illustrate both "diminishing", and "marginal", in the phrase, "diminishing marginal utility". The *marginal* benefits that will be provided, by each additional load of material that is taken, after numerous loads have already been taken, will decrease and diminish, in almost any imaginable system that is adaptive and responsive. In essence, diminishing marginal utility is a restatement of Aristotle's famous phrase, "Moderation in everything."

There are several exceptions and qualifiers to the principle of "diminishing marginal utility". However, in systems that are living, responsive, and adaptable, these exceptions usually operate only at the low end of the scale, and not at the upper end. Examples include systems that require a certain lower limit (often called a "threshold" or "critical mass") to be met and then exceeded, before any substantial benefits will begin to accrue. As one example, when antibiotics are being used to treat a bacterial infection, no good will be accomplished, at all, unless sufficient antibiotics are administered to reduce the bacterial population to a level where a person's immune system can take over and kill the rest. If low and inadequate dosages are used, they are likely to do more damage than good, by creating conditions that will select and promote the growth of antibiotic resistant strains, which will then become even harder to kill.

However, that qualifier operates only at the low end of the curve, and the principle of "diminishing marginal utility" will take over again, once a "useful and effective" threshold is reached and passed. For example, if a week-long dosage of antibiotics, at a useful daily dosage, can help cure an infection, that does not mean a double-dosage will be twice as good for the patient, or that a triple-dosage will do three times as much good. Instead, super-heavy dosages typically do more harm than good, by causing digestive upsets, nausea, blurred visions, or other adverse side effects. The "marginal utility" that is provided by higher and higher dosages of antibiotics (or any other drugs) will decrease and diminish, until it drops quite literally into a negative zone, where additional quantities will cause more harm than good.

The reference above to "systems that are living, dynamic, and inherently responsive and adaptable" includes living organisms and ecosystems, while excluding, for example, structures that must be built in a certain and exact way. For example, if a construction company is building an office building that will be 30 stories tall, the builders will need a certain amount of steel, a certain amount of concrete, etc. On the lower and ascending side of the marginal utility curve, amounts that are less than needed will not be able to do "most of the good"; instead, they will simply leave the building unfinished. On the post-peak descending side of the curve, unneeded surplus amounts will not gradually decrease in value or utility; instead, after the necessary quantity has been reached, the value of any surplus drops almost immediately to zero, like a curve that has fallen off a cliff.

Accordingly, the principle of "diminishing marginal utility" must be understood and applied with regard for its boundaries and limits. However, the boundaries and limits listed above do not apply to mammalian nutrition, since mammals are responsive and adaptive organisms that have evolved over billions of years in ways that enable them to sustain "homeostasis" (this term refers to a responsive and adaptive equilibrium, which keeps operating parameters such as body temperature, blood flow rates, etc., reasonably constant and within functional limits, despite wide variations in surroundings and inputs). These types of homeostatic mechanisms show up fairly quickly, and are quite effective, if a person ingests too much of a certain compound that normally is a good nutrient, but which happens to be received in too great a quantity, on some particular day.

With regard to foods and nutrients, a marginal utility curve is initially steep, since foods and nutrients are necessary to avoid starvation, disease, and death. Then, once a substantial quantity has been ingested and the critical needs of the organism have been met, the marginal utility curve becomes less steep. At some point, the curve will flatten out, and it will reach a peak, hump, or plateau, where the maximal possible good has been achieved by that particular nutrient. After that point, the curve will turn negative, and any additional intake will no longer do any good at all; instead, additional intake beyond the peak level will merely create a waste disposal problem, which the organism will need to take care of, somehow.

That is indeed what happens with excessive zinc ingestion, and the process is controlled by various types of zinc-protein binding activities. In circulating blood, about 80 to 90% of zinc is either inside blood cells, or bound to proteins on the blood cell surfaces. In blood plasma, which contains about 1 to 1.3 *µ*g/mL zinc, the large majority of soluble zinc is reversibly bound to various proteins, including albumin, alpha macroglobulin, and transferrin (e.g., Vallee 1988, Cousins 1989, and Vallee et al 1993). Those proteins provide a "buffer" or "temporary holding" capacity; if more zinc is needed by cells or other systems, proteins such as albumin, alpha macroglobulin, and transferrin can readily release the zinc they are carrying, and allow it to be taken by other proteins, including proteins that will transport the zinc into cell interiors. However, if excess zinc is consumed (such as by people who take large quantities in vitamin supplements), a different process will be triggered. If zinc levels begin to increase beyond the normal homeostatic "set point" in circulating blood, various enzymes and genes will respond in a way that triggers the expression and production of "chelating" proteins, which bind much more tightly to free zinc. These "chelating" proteins notably include metallothionein (Sadhu et al 1990). These tight-binding proteins, secreted by organs such as the kidneys and pancreas in response to increased blood concentrations, help inactivate zinc in two different but complementary ways. Some of these proteins, secreted into the intestines, will bind to zinc while it remains in the intestines, thereby preventing it from crossing the intestinal walls and reaching circulating blood. Other proteins, secreted either into circulating blood or within kidney tissues, will grab zinc ions and transport them across the blood-filtering membranes in the kidneys, thereby causing the excess zinc to be secreted in urine.

The primary route of secretion of zinc is via feces, and fecal concentrations tend to be relatively stable, while urine concentrations fluctuate more rapidly, in response to elevated or reduced blood concentrations. Therefore, elimination in feces provides a fairly stable, constant, steady-state mechanism that handles the bulk of any surplus zinc, while elimination in urine provides a "fine tuning" mechanism that can respond more rapidly to brief fluctuations in blood levels.

Accordingly, the combined actions and principles of homeostasis and diminishing marginal utility offer a crucial and useful opening and opportunity, to help determine and provide optimal zinc dosages for elderly people, who want, need, and deserve help in their efforts to prevent or treat macular degeneration, but who also do not want to get Alzheimer's disease or suffer from aggravated brain damage and permanent impairment if they have a stroke or other crisis.

The fact that a zinc dosage of roughly 80 mg/day was shown to be helpful in slowing the progression of macular degeneration (as shown by the AREDS-1 trial) does not mean or imply, in any way, that a dosage of only half as much zinc would provide only half as many benefits. Instead, because most of the oversupply generated by large zinc dosages will be eliminated in feces and urine, and because of how "diminishing marginal utility" actually works in biology, the much greater probability is that most of the benefits of zinc, in preventing or treating macular degeneration, can be provided by a supplement dosage in the range of about 15 to 30 mg/day. Increasing those dosages to higher but not dangerously high levels, it is likely that the large majority (roughly estimated to be in the range of about 70 to 90 %, or possibly even 95%) of the benefits that might be provided by a 70 to 80 mg daily dosage, in preventing macular degeneration, could be provided by a dosage of only half that much, in a range of 35 to 40 mg/day (which, it must be kept in mind, is still quite a bit higher than most elderly people normally receive each day).

It should be kept in mind that 40 mg/day has been designated as the "Tolerable Upper Intake Level" for zinc, as determined by nutritional experts who extensively studied the biochemistry and physiological effects of zinc. However, it also should be noted that those experts did not address and apparently did not know about the potential neurotoxic risks of zinc, in possibly triggering or accelerating β-amyloid plaques Alzheimer's disease, or in aggravating the extent of brain damage following a stroke or other crisis. Accordingly, the "Tolerable Upper Intake Level" might have been set at a lower level, if not for all adults, then at least for people over the age of about 60 or so, if the experts on the Food and Nutrition Board, at the Institute of Medicine, had known about the apparent connections between zinc and Alzheimer's disease, and zinc and excitotoxic brain damage following a crisis.

In view of all known relevant factors, and unless and until clinical trials are carried out to specifically measure and establish dose-response curves in elderly patients who fall into various nutritional, age, body weight, or other categories, the Applicants herein have concluded that, in eye-care formulations that are marketed primarily to elderly consumers, the following guidelines should be followed:
(1) When taken on a preventive (prophylactic) basis, to sustain good eye and vision health, in the absence of a diagnosed disorder (or a loss of vision clarity that has become apparent to the person), daily dosages of zinc generally should not exceed about 30 mg/day (all dosages herein are expressed as elemental zinc, rather than as zinc oxide, gluconate, etc.). Daily dosages of 40 mg should be regarded as a maximum tolerable level, if supplemented by a copper dosage of about 0.5 to 1.5 mg.
(2) When taken on a therapeutic basis, by a person with a diagnosed eye or vision disorder, or a person who has suffered a noticeable decrease in vision clarity that cannot be corrected by lenses, a series of two different dosage regimens is recommended. The first stage can be regarded as a "rapid buildup" or "crisis response" stage. During that period, which generally will last from about 2 to about 8 weeks, a person with a known eye disorder will need to reach higher blood concentrations of zinc, as quickly as possible. Accordingly, during that stage, the recommended dosage may be in a range of about 30 to about 50 mg/day, depending on body weight (30 mg/day is recommended for an adult with a body weight ranging from about 50 to 60 kilograms, or about 110 to 130 pounds, while higher dosages are recommended for larger and heavier adults). After an initial "rapid buildup" regimen is completed, the person should then take somewhat lower long-term zinc dosages, designed to sustain blood concentrations at levels higher than were present when the eye or vision problem was first noticed. Preferred dosages should be determined by evaluating blood tests, for each specific patient; however, among people who cannot afford such tests (this class is the most likely to suffer from poor nutrition, and it is also the most likely to impose the heaviest burdens on society when people in this class become functionally blind or demented), preferred zinc dosages for long-term ingestion, to help prevent a known vision problem from worsening, generally will be in a range of about 20 to 40 mg/day, depending on age, body weight, and other factors.

In order to prevent the neurologic risks of these zinc dosages from outweighing their advantages in protecting eye and vision health, these dosages of zinc should be accompanied by at least one and preferably two or more types of neuroprotective agents, as discussed below.

It also should be emphasized that the dosage ranges recommended above, for zinc, should be evaluated in clinical trials that also administer zeaxanthin (and preferably β-cryptoxanthin as well) to all test subjects, at dosages of at least 5 and preferably 10 to 20 mg per day. For reasons described below, the Applicants herein believe and assert that zeaxanthin very likely is the true key to preventing and treating macular degeneration, and:
(1) any other agents (including zinc) that are taken to prevent or treat macular degeneration will have only limited and indirect benefits, unless those other agents are accompanied by zeaxanthin supplements;
(2) the best way to determine truly balanced and optimal zinc dosages, for preventing macular degeneration among elderly consumers, is by testing zinc dosages in conjunction with zeaxanthin dosages of at least 5 and preferably at least 10 mg/day; and,
(3) because of factors known to Applicants based on tests on animals and human cadavers, Applicants also believe that zeaxanthin and β-cryptoxanthin enter brain tissue, and probably perform as neuroprotective agents.

### CANDIDATE OCULAR ACTIVE NUTRIENTS

In addition to zeaxanthin, zinc, and vitamins C and E, eight other categories of ocular-active nutrients are listed and briefly described below. Each and all of these eight categories are believed to offer good and promising candidates for early evaluation, to determine whether they can provide synergistic benefits when orally ingested along with zeaxanthin.

It should be recognized that various agents listed below are covered by various patents in the US and elsewhere, including various US patents listed in the Background section. Since various patents may block the lawful inclusion of one or more of these listed nutrients in one or more countries around the world, the nutrient mixtures described in the Examples and claim below have been grouped into baseline components, enhancing ingredients, and optional agents. Zeaxanthin is the baseline component that must be included in any and all such formulations.

Any elderly person who is suffering from ocular problems (or who wants to avoid ocular problems and sustain good eye health) should also take zinc, at a healthy daily dosage or at an elevated therapeutic dosage if he or she suffers from macular degeneration. However, since zinc (including zinc in relatively high dosages) is already contained in numerous oiner products, including multi-vitamins and supplements that are widely taken by elderly people, it must be approached and regarded as an optional agent herein, so that a physician, optometrist, or consumer can properly adjust any zinc content in a formulation as described herein in ways that will not exceed a 40 mg/day total dosage.

At least two (and preferably more, up to and including all) of the "enhancing ingredients" listed herein should also be included, to obtain increased benefits from the ocular supplements disclosed herein.

Optional ingredients are entirely optional, and their desirability and preferred dosages, in ocular supplements as described herein, will depend on whether a consumer is already taken these substances (and at what dosages) in a multi-vitamin product or other supplement.

It also should be noted that many of the compounds mentioned below have one or more "chiral" carbon atoms, and therefore have different stereoisomers. As a general rule, if any one particular stereoisomer is predominant in plant sources or animal tissue, then a strong presumption arises that steps should be taken to provide the natural stereoisomer in a purified or semi-purified form, in any ocular-active nutrient that is being sold or administered to people who wish to protect their eye health. Various known factors suggest that the eye is one of the most "stereo-specific" organs anywhere in the body, and is highly sensitive to differences in stereoisomers. In many cases, this goal can be accomplished by using plant extracts, or by using compounds that have been synthesized by chemically modifying plant-derived stereospecific precursors.

### 1. LIPOIC ACID

This is a fatty acid having 8 carbon atoms in a straight chain, with the carboxy group at the #8 carbon atom, and with the #1 and #3 carbon atoms both coupled to mercaptan groups (-SH, also called sulfhydryl or sulfide groups). In the reduced form, the two mercaptan groups stay separated from each other, with hydrogen protons attached to the sulfur atoms in both pendant groups. In the oxidized form, the hydrogen protons are removed, and the two sulfur atoms bond to each other, to form a five-member ring with the #1, #2, and #3 carbon atoms forming the remainder of the ring.

Because it can convert back and forth between a reduced form and an oxidized form, lipoic acid can help reduce and prevent unwanted oxidation of cells and tissues, and under some circumstances, it can also help regenerate vitamin E (Stoyanovsky et al 1995). Other articles that describe lipoic acid's ability to protect ocular tissues in various tests include Packer 1994, Obrosova et al 1998, Borenshtein et al 2001, Chidlow 2002, and Goralska et al 2003.

Maitra et al 1996 reported that the naturally-occurring "R" (dextrorotatory) stereoisomer has better anti-oxidant activity than the S (levorotatory) isomers that are found in synthetic racemic mixtures. Accordingly, lipoic acid preparations having pure or enriched R stereoisomers are preferred for testing and evaluation as disclosed herein.

### 2. OMEGA-3 FATTY ACIDS

Certain types of compounds that animals must eat in their diets are called "essential fatty acids", because (i) animals need them, mainly for cell membrane formation, but animals cannot synthesize them; (ii) they contain a chain of carbon atoms with a length (usually ranging from about 10 to about 24 carbon atoms) that will form a fatty substance that is solid or semi-solid at room temperature; and (iii) the last carbon atom in the carbon chain is part of a carboxylic acid group (-COOH).

In humans, the three most important essential fatty acids are docosa-hexaenoic acid (abbreviated as DHA), eicosa-pentaenoic acid (EPA), and alpha-linolenic acid (ALA). All three of these compound are called omega-3 fatty acids, since the #3 carbon atom (counting from the non-acid end of the chain) is the first carbon atom that is involved in an unsaturated bond. All three of those omega-3 fatty acids are present in relatively high concentrations in certain types of fish oils, and they can also be obtained from other natural sources, such as certain types of marine algae. They are associated with a number of health benefits, including cardiovascular benefits, anti-cancer activity, etc., so they are of substantial interest throughout the entire field of dietary supplements, as described in articles such as Connor 2000.

Omega-6 fatty acids (with the first double-bond positioned between the #6 and #7 carbon atoms in the carbon chain) are more abundant in nature; however, their health benefits are not as great as for omega-3 fatty acids, and most people already get too many omega-6 fatty acids and not enough omega-3 fatty acids in their diets. Therefore, if a dietary supplement contains a mixture of omega-3 and omega-6 fatty acids, it preferably should contain at least about 30%, and preferably 50% or more, of the omega-3 compounds.

Among the omega-3 fatty acids, DHA has a more important role in mammalian metabolism than EPA, and ALA is generally regarded as merely a precursor to DHA and EPA. Therefore, in purified or semi-purified preparations, DHA is generally the preferred compound, and it has received the most study. Its activities and effects in eyes are described in articles such as Jeffrey et al 2001, Polit et al 2001, Murayama et al 2002, and Rotstein et al 2003.

### 3. PLANT-DERIVED ACTIVE AGENTS (FLAVONOIDS, ANTHOCYANINS, PLANT POLYPHENOLICS, AND PHYTONUTRIENTS)

A third category of candidate ocular-active nutrients that is of interest herein includes a number of plant-derived compounds, which can be referred to by terms that include flavonoids (or bioflavonoids), anthocyanins, plant polyphenolics, or phytonutrients. These labels overlap heavily with each other, and compounds that fall within labels are described in various articles such as Beecher 1999 and Beecher 2003. The molecular structures for each of the named compounds listed below are publicly known, and can be located in various public sources (e.g., the chemical structures of numerous flavonoids, both common and rare, are nicely illustrated and organized at http://www.friedli.com/herbs/phytochem/flavonoids.html).

Compounds that fall within the categories of flavonoids, anthocyanins, plant polyphenolics, or phytonutrients can include either or both of the following: (i) non-purified or semi-purified multi-component mixtures that have been extracted from the fruits, leaves, seeds, nuts, or other parts of various known plants, such as bilberry, grapeseed, green tea, or soybeans; or, (ii) specific known and purified compounds (or limited mixtures of a small number of similar and related compounds) from such plants, such as quercetin, genestein, diazedem, fisetin, luteolin, resveretrol, and pycogenol.

These and various other similar known agents have different specific activities and roles, and each one needs to be considered separately. For example, most flavonoid compounds reduce the activity of an enzyme called aldose reductase. This enzyme converts certain types of beneficial sugars (such as glucose) into sugar-alcohols (such as sorbitol) that will cause problems if they accumulate in excessive quantities. Sorbitol is an important causative factor in cataract formation, especially among diabetics. Therefore, flavonoids that inhibit aldose reductase enzymes can help prevent or slow down cataract formation (e.g., Jung et al 2002, Matsuda et al 2002).

The specific activities, in animal eyes, of any known plant polyphenol (or flavonoid, anthocyanin, phytonutrient, etc.) that has been studied in animals can be identified fairly easily, by searching the free database that is maintained by the National Library of Medicine. As examples, resveretrol reportedly can suppress vascularization (e.g., Brakenhielm et al 2001), and is a good antioxidant and free radical scavenger (Lorenz et al 2003), while genistein reportedly inhibits certain protein kinase enzymes, and can help suppress unwanted types of cell-signalling pathways (e.g., Yoon 2000).

### 4. TAURINE

Taurine is the common name for 2-amino-ethane-sulfonic acid, a "conditionally essential nutrient" that is present in milk and elsewhere. Taurine's ability to protect various ocular tissues in various types of tests (especially involving diabetic pathologies) is described in articles such as Devamanoharan et al 1998, Obrosova et al 1999 and 2001, Chen et al 2000, Militante et al 2002, Pasantes-Morales et al 2002, and DiLeo et al 2003.

### 5. CARNITINE

L-Carnitine is a sulfur-containing amino acid (not one of the 20 primary amino acids used in protein synthesis) that is formed in the liver and certain other tissues. It is believed to facilitate the transport of fatty acids into mitochondria, for certain types of oxidation. Certain esters of carnitine (mainly acetyl-L-carnitine) are preferred for oral ingestion.

Carnitine's ability to help prevent or treat ocular disorders is described in articles such as Pessotto et al 1997, Peluso et al 2001, Alagoz et al 2002, and Feher et al 2003, and in patents such as US 5,753,703 (Cavazza et al 1998). The acetyl-L-carnitine precursor is one of three ingredients (along with omega-3 fatty acids, and coenzyme Q10) in an ocular formulation called PHOTOTROP™, sold by the Sigma Tau Company.

### 6. COENZYME-Q10

An enzyme cofactor known as Coenzyme-Q10 (the Q stands for quinone) is a known anti-oxidant that provides energy-related support to mitochondria. Mitochondria are organelles, inside animal cells, that are enclosed within their own membranes and that have their own set of genes (these genes even use their own special genetic code, which is slightly different from the standard genetic code used in the nucleus of a cell). In a truly remarkable feat of adaptive biology, mitochondria actually are the descendants of tiny anaerobic bacteria, which invaded larger cells billions of years ago, and which then established a symbiotic relationship with their host cells. In this symbiotic relationship, the invaders-turned-guests carry out processes known as "oxidative phosphorylation", which is a crucial part of energy metabolism in the host cells. Because of this role, mitochondria are sometimes referred to as the "furnaces" that handle the burning operations that supply heat and power to the rest of the cell.

When mitochondria are under severe stress, they begin releasing certain types of cytochrome compounds, which will then begin acting as signalling compounds, which will activate a process called "apoptosis", also referred to as "programmed cell death". Apoptosis is a natural process that is beneficial in most situations, since it gives tissues and organs a way to clean up and get rid of dead and dying cells, and replace them with newly-formed and healthy cells. However, in some situations (especially involving neurons, which are extremely difficult and often impossible to replace), apoptosis can lead to severe problems, including (in eye tissues) the unprogrammed and unwanted death and destruction of neurons in the retina. Therefore, by helping stabilize mitochondria, Coenzyme-Q10 can help prevent the release of mitochondrial cytochromes that would lead to unwanted cell deaths, in ocular tissues that are struggling to cope with a serious disorder.

As mentioned above, Coenzyme Q10 is one of the three ingredients in an ocular formulation called PHOTOTROP™, sold by the Sigma Tau Company.

### 7. CARNOSINE

Carnosine is a di-peptide, formed when alanine and histidine bond to each other. It can bond to and quench aldehydes, which are potentially dangerous reactive molecules that can otherwise cause random and unwanted modifications (such as glycosylation or crosslinking) to proteins. The most commonly used orally-ingestible form of carnosine is an ester precursor, N-alpha-acetyl-carnosine. Eyedrops containing carnosine also have been developed and are being publicly sold in Europe.

The protective activities and effects of carnosine in ocular tissues are described in articles such as Maichuk et al 1997, Hipkiss et al 1998, and Babizhayev et al 2002.

### 8. GLUTATHIONE BOOSTERS

Glutathione is a tri-peptide molecule, formed by three amino acids linked together, with cysteine in the middle. Cysteine has a highly reactive sulfur group (-SH) as its side chain. This allows the glutathione tri-peptide to become bonded to other compounds.

With the help of enzymes such as glutathione-S-transferase, glutathione most commonly gets bonded to waste metabolites. This makes the waste products more soluble in water, which in turn helps cells and tissues eliminate those wastes, through pathways that typically end up in urine.

Since the glutathione system provides a useful pathway that helps cells and tissues get rid of waste products, nutrients that can stimulate the production or metabolism of glutathione can help badly-stressed cells and tissues cope more successfully with their waste-handling problems. One such nutrient is N-acetyl cysteine, an ester that when ingested orally will release cysteine, the sulfur-containing amino acid that sits at the center of the glutathione tri-peptide. Other candidates agents that are believed to boost glutathione production or metabolism include selenium, pyridoxine, and riboflavin. These are disclosed, as agents that can help treat macular degeneration, in US patent 5,075,116 (LaHaye 1991).

This completes the summary of the eight categories of agents that offer good candidates for early evaluation along with zeaxanthin. The next two sections describe various factors that need to be taken into account during the design and conduct of animal tests and human clinical trials, which will be necessary to evaluate, quantify, and rank various members of the eight categories of agents listed above, to determine which can offer the best synergistic protection when combined with zeaxanthin.

In addition to the foregoing 8 categories, folic acid (or its ionized form or salt, folate and various folate analogs) also merits consideration, because of its ability to lower levels of a blood compound called homo-cysteine, which is believed to pose a risk factor both in macular degeneration and cardiovascular areas. Preferred dosages for folate generally range from about 400 to 1000 micrograms/day.

### ANIMAL MODELS FOR INITIAL TESTING

As mentioned above, at least five different and distinct animal models are known, for testing candidate ocular-active nutrients. These models include the following:

### 1. Mice and rats, including "knockout" mice

Mice and rats are very widely used in research on small animals, and a huge foundation of information, species-specific biomolecules (including gene promoter sequences, gene coding sequences, monoclonal antibodies, etc.) and specialized strains have been developed for genetic work with mice. Gateways that can be used to access mouse genetic information are freely available on websites such as www.informatics.jax.org and www.ncbi.nlm.nih.gov/genome/seq/MmHome.html. Although the corresponding genetic information on rats is somewhat smaller, it is still enormous and quite useful, and can be accessed through websites such as http://rgd.mcw.edu, http://ratmap.gen.gu.se, and www.hgsc.bcm.tmc.edu/projects/rat.

This genetic information can be put to good use, because a growing number of gene defects have been and are being correlated with known eye disorders. These genes can be discovered by any of several procedures. For example, research revealed that many people who suffer from Stargardt's disease, which causes severe vision impairment have a defective protein known as the Rim protein, which normally functions as an ATP-binding cassette (ABC) transporter gene, in rod outer segment discs, in mammalian retinas. Additional research on that protein (and the gene which encodes that protein) led to identification of a gene called the ABCR gene, as the specific defect that leads to the defective protein in people who suffer from Stargardt's disease. If no properly functioning copies of the ABCR enzyme are present, a toxic metabolite called A2E (shown and described in Radu et al 2003) will gradually accumulate over a span of years in the retinal pigmented epithelial (RPE) layer, directly behind the retina. This accumulating toxin will eventually cause severe damage to the RPE, which is crucial for supporting the retina. As a result, Stargardt's disease usually leads to functional blindness by the time a person reaches the age of about 20.

The most common form of Stargardt's disease is a recessive disorder, which normally occurs only in people who have inherited dysfunctional ABCR genes from both parents (this is referred to as the ABCR -/- genotype). If a person has inherited a properly functioning gene from one parent, and a dysfunctional ABCR gene from the other parent (referred to as the ABCR +/- genotype), Stargardt's disease will not be fully manifested; however, the person will be at elevated risk of serious eye and vision problems later in life.

After the human ABCR gene was identified as a causative factor in Stargardt's disease, a "homologous" ABCR gene in mice was located, which encodes the mouse version of the rim protein. The exact DNA sequence of the mouse ABCR gene was determined, and researchers then used genetic engineering techniques to create mutant mice with "knockout" ABCR genes that are no longer properly functional. These mutant mice, with "knockout" ABCR genes and the mouse equivalent of Stargardt's disease, are described in articles such as Weng et al 1999, Mata et al 2000, Radu et al 2003, and Radu et al 2004. Their descendants suffer from severe visual impairment, which grows gradually worse as certain waste metabolites gradually accumulate within their retinas and retinal pigmented epithelium (RPE) layers. Therefore, the descendants of these knockout mice offer useful animal models, for testing candidate nutrients that may be able to help slow down the gradual accumulation of toxic metabolites in people who have ABCR -/genotypes (leading to Stargardt's disease) or ABCR +/- genotypes (leading to vision problems later in life).

Two additional mouse models of eye diseases that are worth noting include mice that have defective or missing genes that encode proteins referred to as monocyte chemoattractant protein-1 (Ccl-2, also known as MCP-1) and its cognate C-C chemokine receptor-2 (Ccr-2), as discussed in Ambati et al 2003. When those genes are defective, the infiltration of macrophages (specialized types of immune cells) into certain retinal structures (such as the choroidal membrane) is altered, in ways that lead to pathologies that emulate age-related macular degeneration in those retinal tissues.

Yet another mouse model is provided by mice that have defective or missing genes that encode apolipoprotein-E, which is essential for making a properly-functioning Bruch's membrane behind the retina, as discussed in articles such as Dithmar et al 2000 and 2001 and Miceli et al 2000.

These are three specific examples of known gene defects that lead to retinal pathologies, and each of these defects has been used to create an animal model that can be used to test and evaluate the potency of various candidate agents that may be able to help prevent or treat macular degeneration or other retinal pathologies in humans. Other useful animal models are being developed and published by other research teams, and additional information is available in review articles such as Ambati et al 2003.

This work is being expanded and accelerated greatly by various known genetic engineering methods, and by using and comparing gene sequence information that already has been gathered and published as part of the human genome project, the mouse genome project, and the rat genome project. Dozens or even hundreds of genes that express specific proteins involved in eye structures and/or vision processing have been identified, and each such discovery involving a specific gene provides a potentially useful new and distinct animal model for testing.

Four presumptions apply to such research: (1) every structural protein that is present in any eye structure, and every enzymatic protein that is involved in any step in vision processing in the eyes, is present within the eyes for a good reason, and plays some useful and necessary role in vision; (2) a gene defect that renders any such protein nonfunctional will very likely lead to some type of identifiable and potentially important eye disorder; (3) once any such genetic defect has been identified, either in humans or in mice or rats, colonies of lab animals that carry that genetic defect can be created and/or raised; and, (4) any such colony can provide an animal model that can help researchers evaluate and rank the ability of various candidate nutrients or other treatments to overcome the problem that is caused or aggravated by that particular defective gene or protein, in that animal model.

Accordingly, genetic analysis and research, including research involving mice or rat colonies having "knockout" genes that are correlated with specific vision disorders, offer extremely powerful tools, and can provide an effectively unlimited number and range of specific targeted "models" that can help researchers test candidate nutrients, to evaluate whether any nutrient or nutrient combination can act synergistically with zeaxanthin, to help prevent or treat one or more specific types of ocular disorders.

### 2. Use of agents to increase carotenoid uptake in rodents

When carrying out vision-related research on mice or rats, it must be noted that most rodents are prey rather than predators, and almost never go out into direct sunlight in the middle of the day, since that would make them highly vulnerable to predators. Accordingly, rodents did not evolve with any need for carotenoids to help protect them against UV radiation. Therefore, rodents generally do not metabolize carotenoids in ways comparable to humans, and they tend to make relatively poor models for studying the uptake or effects of carotenoids.

However, various manipulations can be used to increase carotenoid uptake in rats and other rodents. As one example, if relatively high concentrations of bile salts or other compounds that help solubilize fatty compounds are added to the diets of mice or rats, the animals will transport higher quantities of carotenoids through the intestinal walls and into circulating blood, which will lead to greater rates and concentrations of tissue deposition. Therefore, by feeding special diets to mice or rats, various types of research involving zeaxanthin (or other carotenoids) can be carried out in these animals.

It should also be recognized that research which directly uses and includes zeaxanthin will not always be necessary, to do research on mice or rats that can help evaluate and rank candidate nutrients that may be able to work synergistically with zeaxanthin. Instead, the benefits of working with mice or rats usually are limited to initial research, which hopefully will lead to expanded and more expensive research on larger animals and/or humans. Accordingly, mice and rats may be well-suited for evaluating candidate nutrients such as lipoic acid, isoflavonoids, plant polyphenols, omega-3 fatty acids, taurine, carnitine, etc., to evaluate their effects on ocular or vision defects, in tests that will not use or include any zeaxanthin or other carotenoids. Subsequently, after initial evaluations and rankings have been determined by means of initial testing in mice or rats, the most promising candidates can then be tested in more expensive tests that will involve zeaxanthin, using animals that metabolize carotenoids in a manner comparable to humans (such as Japanese quails or other suitable birds, or primates), or in human clinical trials.

It should also be recognized that mice, rats, and other rodents do not have pigmented maculas; instead, in general, the only animals that use UV-absorbing carotenoids to protect their retinas are primates, and some species of birds. However, if rats are induced (by bile salts in their diets) to begin taking up substantial quantities of carotenoids into circulating blood, at least some of those carotenoids will be deposited into photoreceptors in the retina, and into the lens of the eye, thereby allowing at least some types of research on those structures.

### 3. Agents and methods to create and emulate disorders

Additional options that can be used to evaluate candidate ocular-active nutrients involves the use of certain drugs or diets, to induce certain types of damage that can emulate known ocular disorders. As one example, cataracts can be induced by a drug called buthionin sulfoximine (e.g., Maitra et al 1996), or by feeding lab animals certain types of high-starch diets (e.g., Borenshtein et al 2001). As another example, diabetes can be induced by drugs such as streptozotocin (e.g., Kowluru et al 2003) or allosan.

If the goal of a research project is to study a disorder that involves abnormally high levels of cell growth (such as wet macular degeneration, with excessive blood vessel growth, or certain types of "proliferative retinopathies"), pellets contain cell-stimulating hormones can be implanted into an eye. Such research, using "vascular endothelial growth factor" (VEGF) or "basic fibroblast growth factor" (bFGF), is described in articles such as Yoon et al 2000 and Joussen et al 2000.

Various types of surgical or mechanical interventions can also be used to emulate certain ocular disorders. As one example, clamping off an artery for a fixed period of time is used to create ischemia, then the clamp can be suddenly released, to create a "reperfusion" injury involving oxygen free radicals.

In addition, external methods can be used to accelerate certain types of visual impairment. Such methods include, for example, increasing the intensity of ultraviolet and blue light, and increasing the atmospheric oxygen concentrations, in the pens or rooms where lab animals are being kept.

Any of these methods can impose additional levels of ocular stress on lab animals, thereby substantially accelerating the rates at which they will develop ocular disorders. Accordingly, various candidate ocular-active nutrients can be evaluated for potency and efficacy, by measuring how effectively they can delay, prevent, or reduce the disorders that will arise from the stresses that were imposed on the animals.

### 4. Japanese quail and other birds

As mentioned above, some types of birds use carotenoid pigments to help protect their retinas against damage by UV light. In most bird species, these pigments are deposited throughout the entire retina, rather than just in a small central area comparable to the maculas of primates. A review of the use of birds, in retinal research, is contained in Fite et al 1991.

Japanese quail have become a widely used and accepted bird model for retinal testing, as described in articles such as Fite et al 1993, Fite 1994. Detailed methods for testing this species, to evaluate the ability of zeaxanthin or lutein to protect against retinal damage caused by high-intensity lights, were described in Thomson et al 2002.

In addition, an albino strain of Japanese quail has been developed, which suffers from rapid lens degeneration and cataract formation.

### 5. Testing of dogs and livestock

Among the types of lab animals larger than rodents that are used in vision testing, dogs and livestock tend to be used most commonly, for various reasons.

With respect to dogs, their irises (which are circular) are more similar to human and primate irises, than the vertical-slit irises of cats; in addition, dogs also suffer fairly commonly from cataracts. They can also be induced to incur various types of retinopathies, and there are certain aspects of their vision processing that are of interest to neurology researchers (including limitations in the ability of dogs to generate nerve impulses that will help them recognize and identify things, unless some type of motion is involved that will trigger a set of nerve cell firings). For all of these reasons, dogs are used fairly commonly for ocular and vision research. While they are more expensive than mice or rats, they are less expensive than primate studies or human clinical trials. Accordingly, if dogs are being considered as a potential animal model for studies as disclosed herein, a network of experts who are already familiar with that type of research in dogs can be located, quickly and easily, by a search for published articles describing vision research in dogs.

Research on eye components or other tissues from various livestock species (including pigs, cows, and sheep) is enabled by an important factor: these animals are killed, in large numbers, at known locations and under controlled conditions (i.e., at slaughterhouses). Therefore, specialized treatment procedures can be carried out on livestock animals shortly before they are killed, and the affected tissues can be harvested at a controlled time, soon thereafter. Alternately, other types of specialized procedures can be carried out on tissue that was harvested immediately after an animal is killed; these types of tissue samples are usually perfused (i.e., placed in specialized equipment that will pump fluids with oxygen and nutrients through or around the tissue), to sustain the tissue in a condition where its cells remain viable and metabolically active for a span of hours or days after the animal was killed. Compared to ocular tissue samples from mice or rats, ocular tissues from animals such as cows or pigs are much easier to handle and work with, and they also provide more relevant results, if dimensional factors are important (such as, for example, when the permeation of a drug or nutrient into or through lens tissue is important).

### 6. Primate tests

Primates include lemurs, monkeys, and apes. While they are expensive to raise, keep, and test, they nevertheless provide animal models that, in some situations, will provide better and more applicable and relevant data than any other type of animal test, short of a human clinical trial. Therefore, they must be kept in mind as one option. In many situations, to keep costs under control, it may be possible to "piggyback" a vision-related test on top of some other type of ongoing test (such as a cancer-related test), using the same animals that are being tested for other purposes.

### HUMAN CLINICAL TRIALS AND META-TRIALS

Based on the results of animal tests, as described above and as otherwise known to those skilled in the art, candidate formulations that have performed well in such animal tests can be further evaluated, in clinical trials. As used herein and in common practice, the term "clinical" implies that the subjects will be humans, rather than laboratory animals.

Proper and lawful general procedures for carrying out human clinical trials are described in numerous published articles and books, and are known to thousands of researchers, consultants, and other experts. Those general procedures and requirements will not be discussed or analyzed herein.

However, two aspects of such testing on humans deserve special note and consideration herein.

The first special point worth noting is this: at least some types of ocular or vision-related tests can be designed to speed up the gathering of useful data, when testing patients who are suffering from diseases that gradually manifest or grow worse over a long span of time, such as multiple years. This type of accelerated data gathering can be enabled by various approaches, such as by focusing on selected patients who, at the point in time when they will be tested, are entering or progressing through certain stages that involve accelerated and rapid degeneration and loss of vision acuity.

As one example, among most patients who suffer from the dry form of macular degeneration (which includes roughly 90% of all cases of macular degeneration), their retinas will pass, at some point during the disease, through an intermediate stage called "geographic atrophy". During this stage, distinct patches and areas of degeneration in or around the macula become visible (as indicated by certain types of cellular debris, such as abnormally large pieces of drusen and lipofuscin), in certain types of photographs that can be taken of the retina.

When retinas suffering from dry macular degeneration reach this stage, and begin to suffer from "geographic atrophy" showing clear and distinct patches of degeneration, they have begun (or will soon begin) to suffer from accelerated and rapid retinal degeneration. Briefly, this process can be depicted, in a schematic manner, by using an "S-curve" that descends from a relatively horizontal plateau, to a lower yet relatively horizontal level (which, in most cases of geographic atrophy, represents functional blindness).

Stated in another way, a person suffering from the dry form of macular degeneration typically will spend years, passing through slow, gradual, and incremental losses of visual acuity, sometimes without even noticing that his or her vision is slowly growing worse (and sometimes choosing to remain silent when they do notice it, for fear of being ordered to stop driving). This long slow stage is represented by the plateau on the left side of the "S curve", as the horizontal time scale goes from left to right.

At some point in time, most victims of macular degeneration will reach a stage when the accumulating stresses seem to begin piling on top of each other, and the person begins to lose visual acuity at an accelerated rate that can no longer be ignored or hidden. When this occurs, if the patient visits an ophthalmologist and has his or her eyes checked, he or she often will be found to be in the rapid descent stage that is characterized by the retinal lesion patterns that are called "geographic atrophy. If effective steps are not taken to halt the spread of the damage, it usually will begin accelerating even more rapidly, and will lead to severe loss of vision, usually ending in functional blindness.

When it comes to clinical testing of candidate ocular-active nutrients, patients who are approaching or who have entered the "rapid decline" stage of degeneration and geographic atrophy can be highly useful and helpful, for carrying out tests that are specially designed to provide relatively rapid data, to help reveal which particular nutrients (out of various candidates being tested) can be the most effective in preventing further degeneration, when combined with zeaxanthin in orally-ingestible formulations and foodstuffs. Accordingly, anyone who is contemplating or designing tests on various candidate ocular-active nutrients, should be alert to the possibility of placing patients who are at the "geographic atrophy" stage of macular degeneration (or at a comparable stage of any other ocular disorder) into a special testing or control population, which can then be analyzed carefully over a shorter period of time than would otherwise be required.

Another important approach that should be considered by anyone contemplating or designing tests on candidate ocular-active nutrients, involves tests usually referred to as "meta-trials". In general, these types of tests involve numerous relatively small data-gathering centers, which are grouped or tied together in ways that allow the data from all of the multiple small centers to be compiled into a larger pool of consistent shared data.

As an example, one of the most promising approaches to human testing of various candidate ocular-active formulations as disclosed herein can use a network of cooperating optometrists and/or ophthalmologists, who are already skilled in examining eyes. Any optometrist or ophthalmologist who wishes to become involved in a meta-trial will need to be instructed (with video, written, or in-person instruction or training, as necessary) in the exact procedures that will need to be followed by all patients enrolled in a test, and by any clerical or healthcare workers who will monitor and review the data gathered at that site.

The procedures that will be used can involve either double-blinded trials, or open-label trials, depending on the desires and goals of the people, companies, or agencies who are organizing and running the study. Monitoring of results can involve any appropriate data-gathering methods, such as visual acuity tests by optometrists (which usually measure "lines of resolution" on standardized eye charts), or more complicated tests by ophthalmologists (such as measurements of pigment densities in lenses or maculas).

If a meta-trial approach is used, each participating optometrist or ophthalmologist will be responsible for gathering data at his or her site, while one or more workers at a coordinating office will be responsible for (i) creating reporting forms that will help ensure that the data received from different sites are uniform and consistent, and (ii) monitoring the quality of the data coming from numerous sites. Participating optometrists or ophthalmologists will be supplied with consistent and exact formulations by a single coordinating office, and if a trial is double-blinded, these products can be in the form of number-coded bottles, containing capsules or tablets that do not indicate whether the contents are test compounds, or controls. Presumably, any such controls likely will contain an anti-oxidant formulation that already has been shown to work at some level of efficacy, such as the AREDS-1 formulation, which contains high dosages of vitamins C and E, beta-carotene, and zinc.

As an illustration of how meta-trials can work, if fifty optometrists or ophthalmologists (each continuing to work out of his or her normal office) are involved, and if each participating optometrist or ophthalmologist enrolls twenty patients in a control group, and twenty patients in a test group, then the combined efforts will generate totals of 1000 patients in the control group, and 1000 patients in the test group.

This approach can be used to generate relatively rapid yet statistically powerful data, without placing a huge burden on any one particular person or location. Accordingly, meta-trials deserve careful attention, since they offer highly promising and relatively rapid yet relatively inexpensive methods for carrying out human clinical trials, involving large numbers of test and control subjects, on candidate ocular-active combinations as described herein.

### EXAMPLES

### EXAMPLE 1: IMPROVEMENTS OVER THE AREDS FORMULA

The nutritional formulation disclosed in this example offers substantial improvements (in both efficacy and safety) over any and all AREDS-formulation supplements that currently are being sold in the United States or elsewhere. The daily dosages listed below can be provided by capsules (which can have granular ingredients and/or an oily carrier liquid), tablets (which can be coated), liquids, or powders, such as in a dosage regimen of 2 or 3 capsules per day, taken with meals. All dosages are specified as recommended daily dosages.
Zeaxanthin: at least 3 mg, preferably 5 mg, even more preferably 10 to 50 mg
Vitamin C: at least 200 mg, up to 2000 mg, preferably 500 mg
Mixed tocopherols: at least 200 international units (IU), preferably 300 to 1000 IU
Zinc: at least 10 mg, preferably 15 to 40 mg but no higher than 40 mg

This mixture is also characterized by the absence of any copper or beta-carotene. Any other ocular nutrients described herein (such as lipoic acid, Co-Q10, taurine, lutein, etc.) are optional.

### EXAMPLE 2: HIGH-DOSAGE FORMULAS FOR TREATING KNOWN PROBLEMS

A strongly effective and beneficial formulation, suited for treating known and ongoing ocular problems, can be provided by including each of the ingredients listed below, at the dosage ranges that are indicated. However, since various patents may block the lawful inclusion of one or more of these listed nutrients in one or more countries around the world, the nutrients listed below have been grouped into "baseline components", "enhancing ingredients", and "optional agents". All baseline components should be included, and at least two and preferably more of the "enhancing ingredients" should also be included. Optional ingredients are entirely optional, and their desirability and preferred dosages will depend on whether a consumer is already taken these substances in a multi-vitamin product or other supplement. All dosages are specified on a daily basis, and may be in forms such as capsules, tablets, liquids, or powders as described in Example 1.

The required "baseline component" is zeaxanthin, at a dosage of least 10 mg, up to 50 mg, for therapeutic (as distinct from preventive) purposes.

In addition, at least two and preferably more (up to and including all) of the following "enhancing ingredients" must also be included, in the dosages indicated below, in a formulation that is covered by the teachings and claims herein:
DHA: at least 100 mg, preferably up to 2000 mg
alpha-R-lipoic acid: at least 25 mg, preferably up to 200 mg
N-acetyl cysteine: at least 100 mg, preferably up to 1000 mg
Co-Q10: at least 10 mg, preferably up to 100 mg
Isoflavones, polyphenols, or and/or other plant extracts: at least 20 mg, preferably 50 to 1000 mg
Acetyl-L-carnitine: at least 50 mg, preferably up to 500 mg

In addition, various "optional agents" should be taken in the dosages suggested below, by any person who is suffering from known and ongoing age-related eye problems. However, since most or all of these agents are already present in numerous other multi-vitamins and other supplements, such other sources must be taken into account:
Zinc: at least 10 mg, preferably 15 to 40 mg but no higher than 40 mg
Vitamin C: at least 200 mg, up to 2000 mg, preferably 500 mg
Mixed tocopherols: at least 200 international units (IU), preferably 300 to 1000 IU
Beta-carotene: 1000 to 5000 IU (and possibly less than 500, for a formulation labelled for smokers)
Copper: up to 1 mg
Selenium: 25 to 100 micrograms
Folic acid or folate: 400 to 1000 micrograms/day

### EXAMPLE 3: FORMULAS FOR PREVENTING PROBLEMS AND PRESERVING EYE HEALTH

A highly beneficial formulation for helping preserve good eye health and good vision in people who are not actively suffering from known and ongoing ocular problems can be provided by ingesting each of the ingredients listed below, at the dosage ranges that are indicated. Since various patents may block the lawful inclusion of one or more of these listed nutrients in one or more countries around the world, the nutrients listed below have been grouped into "baseline components", "enhancing ingredients", and "optional agents". All baseline components should be included and at least two and preferably more of the "enhancing ingredients" should also be included. Optional ingredients are entirely optional, and their desirability and preferred dosages will depend on whether a consumer is already taken these substances in a multi-vitamin product or other supplement. All dosages are specified on a daily basis, and may be in forms such as capsules, tablets, liquids, or powders as described in Example 1.

The required "baseline component" is zeaxanthin, at a dosage of least 1 mg, preferably at least 3 mg, and even more preferably 5 to 20 mg.

In addition, at least two and preferably more (up to and including all) of the following "enhancing ingredients" should also be taken by any consumer who is suffering from known and ongoing age-related eye problems:
DHA: at least 100, preferably 500 to 1000 mg
alpha-R-lipoic acid: at least 25 mg, preferably up to 50 mg
N-acetyl cysteine: at least 50 mg, preferably up to 300 mg
CoQ10: at least 10 mg, preferably up to 30 mg
Isoflavones, polyphenols, or and/or other plant extracts: at least 10 mg, preferably up to 1000 mg
Acetyl-L-carnitine: at least 30 mg, preferably up to 100 mg

In addition, various "optional agents" should be taken in the dosages suggested below, by any person who is suffering from known and ongoing age-related eye problems. However, since most or all of these agents are already present in numerous other multi-vitamins and other supplements, such other sources must be taken into account:
Zinc: at least 10 mg, preferably 15 to 40 mg but no higher than 40 mg
Vitamin C: at least 200 mg, up to 1000 mg, preferably 500 mg
Mixed tocopherols: at least 200 international units (IU), preferably 300 to 400 IU
Beta-carotene: 1000 to 5000 IU (and possibly less than 500, for a formulation labelled for smokers)
Copper: up to 0.5 mg
Selenium: 25 to 100 micrograms
Folic acid or folate: 400 to 1000 micrograms/day

Thus, there has been shown and described a new and useful set of nutritional supplements for preserving ocular health, and for treating ocular disorders.

### REFERENCES

Alagoz G, et al, "L-carnitine in experimental retinal ischemia-reperfusion injury," Ophthalmologica. 2002 Mar-Apr;216(2):144-50
Ambati, J., et al, "An animal model of age-related macular degeneration in senescent Ccl-2 or Ccr-2-deficient mice," Nat Med 9: 1390-97 (2003)
Ambati, J., et al, "Age-related macular degeneration: etiology, pathogenesis, and therapeutic strategies," Surv Ophthamol 48: 257-93 (2003)
Areias FM, et al, "Antioxidant effect of flavonoids after ascorbate/Fe(2+)-induced oxidative stress in cultured retinal cells," Biochem Pharmacol. 2001 Jul 1;62(1):111-8
Armstrong, D., et al, "Lipid hydroperoxide stimulates retinal neovascularization in rabbit retina through expression of tumor necrosis factor-alpha, vascular endothelial growth factor and platelet-derived growth factor," Angiogenesis 2(1): 93-104 (1998)
Babizhayev MA, et al, "Efficacy of N-acetylcarnosine in the treatment of cataracts," Drugs R D. 2002;3(2):87-103
Beaumont P., "Zinc and macular degeneration, Arch Ophthalmol. 111: 1023 (1993)
Beecher GR, "Phytonutrients' role in metabolism: effects on resistance to degenerative processes," Nutr Rev. 1999 Sep;57(9 Pt 2):S3-6
Beecher GR, "Overview of dietary flavonoids: nomenclature, occurrence and intake," J Nutr. 2003 Oct;133(10):3248S-3254S
Borenshtein D, et al, "Cataract development in diabetic sand rats treated with alpha-lipoic acid and its gamma-linolenic acid conjugate," Diabetes Metab Res Rev. 2001 Jan-Feb;17(1):44-50
Brakenhielm E, et al, "Suppression of angiogenesis, tumor growth, and wound healing by resveratrol, a natural compound in red wine and grapes," FASEB J. 2001 Aug;15(10):1798-800
Bush AI, et al, "Rapid induction of Alzheimer Abeta amyloid formation by zinc," Science. 1994 Sep 2;265(5177): 1464-7
Bush AI, "Metal complexing agents as therapies for Alzheimer's disease," Neurobiol Aging. 2002 Nov-Dec; 23(6): 1031-8.
Canzoniero LM, et al, "Membrane-permeant chelators can attenuate Zn2+-induced cortical neuronal death," Neuropharmacology. 2003 Sep;45(3): 420-8.
Cao Y, et al, "Antiangiogenic mechanisms of diet-derived polyphenols," J Nutr Biochem. 2002 Jul;13(7):380-390
Carpenter, K.L., et al, "The carotenoids beta-carotene, canthaxanthin and zeaxanthin inhibit macrophage-mediated LDL oxidation," FEBS Lett 401(2-3): 262-6 (1997)
Castillo M, et al, "Effects of hypoxia on retinal pigmented epithelium cells: protection by antioxidants," Ophthalmic Res. 2002 Nov-Dec;34(6):338-42
Chaney MO, et al, "Abeta, aging, and Alzheimer's disease: a tale, models, and hypotheses," Neurol Res. 2003 Sep;25(6): 581-9
Chen F, et al, "An experimental research of taurine on H2O2-induced bovine lens epithelial cell apoptosis," Zhonghua Yan Ke Za Zhi. 2000 Jul;36(4):272-4, 17
Cherny RA, et al, "Aqueous dissolution of Alzheimer's disease Abeta amyloid deposits by biometal depletion," J Biol Chem. 1999 Aug 13;274(33): 23223-8
Cherny RA, et al, "Treatment with a copper-zinc chelator markedly and rapidly inhibits beta-amyloid accumulation in Alzheimer's disease transgenic mice," Neuron. 2001 Jun;30(3): 665-76
Chew, B.P., et al, "Dietary lutein inhibits mouse mammary tumor growth by regulating angiogenesis and apoptosis," Anticancer Res 23(4): 3333-9 (2003)
Chidlow G, et al, "Alpha-lipoic acid protects the retina against ischemia-reperfusion," Neuropharmacology. 2002 Nov;43(6):1015-25
Choi DW, Koh JY, "Zinc and brain injury," Annu Rev Neurosci. 1998;21: 347-75.
Connor WE., "Importance of omega-3 fatty acids in health and disease," Am J Clin Nutr. 2000 Jan;71(1 Suppl) :171S-5S
Cuajungco MP, et al, "Metal chelation as a potential therapy for Alzheimer's disease," Ann N Y Acad Sci. 2000;920: 292-304.
Cuajungco MP, Faget KY, "Zinc takes the center stage: its paradoxical role in Alzheimer's disease," Brain Res Brain Res Rev. 2003 Jan;41(1): 44-56.
Devamanoharan PS, et al, "Oxidative stress to rat lens in vitro: protection by taurine," Free Radic Res. 1998 Sep;29(3):189-95
DiLeo MA, et al, "Potential therapeutic effect of antioxidants in experimental diabetic retina: a comparison between chronic taurine and vitamin E plus selenium supplementations," Free Radic Res. 2003 Mar;37(3):323-30
Dithmar, S., et al, "Ultrastructural changes in Bruch's membrane of apolipoprotein E-deficient mice," Invest Opthalmol Vis Sci 41: 2035-42 (2000)
Dithmar, S., et al, "Murine high-fat diet and laser photochemical model of basal deposits in Bruch membrane," Arch Opthamol 119: 1643-9 (2001)
Erlund I, et al, "Consumption of black currants, lingonberries and bilberries increases serum quercetin concentrations," Eur J Clin Nutr. 2003 Jan;57(1):37-42
Feher J, et al, "Mitotropic compounds for the treatment of age-related macular degeneration. The metabolic approach and a pilot study," Ophthalmologica. 2003 Sep-Oct;217(5):351-7
Finefrock AE, et al, "Current status of metals as therapeutic targets in Alzheimer's disease," J Am Geriatr Soc. 2003 Aug;51(8): 1143-8.
Ford, E.S., et al, "C-reactive protein concentration and concentrations of blood vitamins, carotenoids, and selenium among United States adults," Eur J Clin Nutr 57(9): 1157-63 (2003)
Frederickson CJ, Bush AI, "Synaptically released zinc: physiological functions and pathological effects," Biometals. 2001 Sep-Dec;14(3-4): 353-66.
Friedlich AL, et al, "Neuronal zinc exchange with the blood vessel wall promotes cerebral amyloid angiopathy in an animal model of Alzheimer's disease, J Neurosci. 2004 Mar 31;24(13): 3453-9
Gaynes BI, "AREDS misses on safety," Arch Ophthalmol. 2003 Mar;121(3): 416-7
Gonzalez, S., et al, "Dietary lutein/zeaxanthin decreases ultravoilet B-induced epidermal hyperproliferation and acute inflammation in hairless mice," J Invest Dermatol 121(2): 399-405 (2003)
Goralska M, et al, "Alpha lipoic acid changes iron uptake and storage in lens epithelial cells," Exp Eye Res. 2003 Feb;76(2):241-8
Hawkins WR., "Zinc supplementation for macular degeneration," Arch Ophthalmol. 109: 1345 (1991)
Hipkiss AR, et al, "Pluripotent protective effects of carnosine, a naturally occurring dipeptide," Ann N Y Acad Sci. 1998 Nov 20;854:37-53
Jampol LM, "Antioxidants, zinc, and age-related macular degeneration: results and recommendations," Arch Ophthalmol. 2001 Oct;119(10): 1533-4
Jampol LM, "AREDS-two years later," Arch Ophthalmol. 2003 Nov;121(11): 1634-6
Jeffrey BG, et al, "The role of docosahexaenoic acid in retinal function," Lipids. 2001 Sep;36(9):859-71
Joussen AM, et al, "Treatment of corneal neovascularization with dietary isoflavonoids and flavonoids," Exp Eye Res. 2000 Nov;71(5):483-7
Jung SH, et al, "Isoflavonoids from the rhizomes of Belamcanda chinensis and their effects on aldose reductase and sorbitol accumulation in streptozotocin induced diabetic rat tissues," Arch Pharm Res. 2002 Jun;25(3):306-12
Kagan BL, et al, "The channel hypothesis of Alzheimer's disease: current status," Peptides. 2002 Jul;23(7): 1311-5.
Kahkonen MP, et al, "Berry phenolics and their antioxidant activity," J Agric Food Chem. 2001 Aug;49(8):4076-82
Kar S, Quirion R, "Amyloid beta peptides and central cholinergic neurons: functional interrelationship and relevance to Alzheimer's disease pathology," Prog Brain Res. 2004;145: 261-74.
Kilic F, et al, "Modelling cortical cataractogenesis XX. In vitro effect of alpha-lipoic acid on glutathione concentrations in lens in model diabetic cataractogenesis," Biochem Mol Biol Int. 1998 Oct;46(3):585-95
Kocer I, et al, "Protection of the retina from ischemia-reperfusion injury by L-carnitine in guinea pigs," Eur J Ophthalmol. 2003 Jan-Feb; 13(1):80-5
Kowluru RA, et al, "Diabetes-induced mitochondrial dysfunction in the retina," Invest Ophthalmol Vis Sci. 2003 Dec;44(12):5327-34
Kowluru RA., "Diabetes-induced elevations in retinal oxidative stress, protein kinase C and nitric oxide are interrelated," Acta Diabetol. 2001 Dec;38(4): 179-85
Lee, E.H., et al, "Dietary lutein reduces ultravoilet radiation-induced inflammation and immunosuppression," Invest Dermatol 122(5): 510-7 (2004)
Lee, S.J., et al, "Axtaxanthin inhibits nitric oxide production and inflammatory gene expression by suppressing I(kappa)B kinase-dependent NF-kappaB activation," Mol Cells 16(1): 97-105 (2003)
Lee JM, et al, "Zinc translocation accelerates infarction after mild transient focal ischemia," Neuroscience. 2002;115(3): 871-8.
Leitzmann MF, et al, "Zinc supplement use and risk of prostate cancer," J Natl Cancer Inst. 2003 Jul 2;95(13): 1004-7
Lobner D, et al "Zinc-induced neuronal death in cortical neurons," Cell Mol Biol (Noisy-le-grand). 2000 Jun;46(4): 797-806.
Lorenz P, et al, "Oxyresveratrol and resveratrol are potent antioxidants and free radical scavengers: effect on nitrosative and oxidative stress derived from microglial cells," Nitric Oxide. 2003 Sep;9(2):64-76
Maichuk IF, et al, "[Development of carnosine eyedrops and assessing their efficacy in corneal diseases] Vestn Oftalmol. 1997 Nov-Dec;113(6):27-31
Maitra I, et al, "Stereospecific effects of R-lipoic acid on buthionine sulfoximine-induced cataract formation in newborn rats," Biochem Biophys Res Commun. 1996 Apr 16;221(2):422-9
Manzanas L, et al, "Oral flavonoids, chromocarb diethylamine salt and cyaninosides chloride, to eliminate lipoperoxidation postvitrectomy," Exp Eye Res. 2002 Jan;74(1):23-8
Manzerra P, et al, "Zinc induces a Src family kinase-mediated up-regulation of NMDA receptor activity and excitotoxicity," Proc Natl Acad Sci U S A. 2001 Sep 25;98(20): 11055-61.
Matsuda H, et al, "Structural requirements of flavonoids and related compounds for aldose reductase inhibitory activity," Chem Pharm Bull (Tokyo). 2002 Jun;50(6):788-95
Miceli, M.V., et al, "Pathologic changes in the retinal pigment epithelium and Bruch's membrane of fat-fed atherogenic mice," Curr Eye Res 20: 8-16 (2000)
Militante JD, et al, "Taurine: evidence of physiological function in the retina," Nutr Neurosci. 2002 Apr;5(2):75-90
Moyad MA, "Zinc for prostate disease and other conditions: a little evidence, a lot of hype, and a significant potential problem," Urol Nurs. 2004 Feb;24(1): 49-52
Murayama K, et al, "Fish oil (polyunsaturated fatty acid) prevents ischemic-induced injury in the mammalian retina," Exp Eye Res. 2002 Jun;74(6):671-6
Newsome, D.A., et al, "Oral zinc in macular degeneration," Arch. Ophthalmol. 106: 192-198 (1988)
Newsome, DA, et al, "Oral zinc in macular degeneration," Arch Ophthalmol 1988; 106: 192-198.
Obrosova IG, et al, "Effect of dietary taurine supplementation on GSH and NAD(P)-redox status, lipid peroxidation, and energy metabolism in diabetic precataractous lens," Invest Ophthalmol Vis Sci. 1999 Mar;40(3):680-8
Obrosova IG, et al, "Taurine counteracts oxidative stress and nerve growth factor deficit in early experimental diabetic neuropathy," Exp Neurol. 2001 Nov;172(1):211-9
Obrosova I, et al, "Diabetes-induced changes in lens antioxidant status, glucose utilization and energy metabolism: effect of DL-alpha-lipoic acid," Diabetologia. 1998 Dec;41(12): 1442-50
Obrosova IG, et al, "Early changes in lipid peroxidation and antioxidative defense in diabetic rat retina: effect of DL-alpha-lipoic acid," Eur J Pharmacol. 2000 Jun 9;398(1): 139-46
Ohgami, K., et al, "Effects of astaxanthin on lipopolysaccharide-induced inflammation in vitro and in vivo," Invest Ophthamol Vis Sci 44(6): 2694-701 (2003)
Okuyama H, et al, "alpha-linolenate-deficiency-induced alterations in brightness discrimination learning behavior and retinal function in rats," World Rev Nutr Diet. 2001;88:35-40
Packer L., "Antioxidant properties of lipoic acid and its therapeutic effects in prevention of diabetes complications and cataracts," Ann N Y Acad Sci. 1994 Nov 17;738:257-64
Pasantes-Morales H, et al, "Treatment with taurine, diltiazem, and vitamin E retards the progressive visual field reduction in retinitis pigmentosa: a 3-year follow-up study," Metab Brain Dis. 2002 Sep;17(3):183-97.
Peluso G, et al, "Carnitine protects the molecular chaperone activity of lens alpha-crystallin and decreases the post-translational protein modifications induced by oxidative stress," FASEB J. 2001 Jul;15(9):1604-6
Pessotto P, et al, "In experimental diabetes the decrease in the eye of lens carnitine levels is an early important and selective event," Exp Eye Res. 1997 Feb;64(2):195-201
Polit L, et al, "Effects of docosahexaenoic acid on retinal development: cellular and molecular aspects," Lipids. 2001 Sep;36(9):927-35
Pulido JS, Blake CR, "Special considerations in the guidelines for high-dose vitamin supplementation in patients with age-related macular degeneration," Arch Ophthalmol. 2004 Apr; 122(4): 662
Ritchie CW, et al, "Metal-protein attenuation with iodochlorhydroxyquin (clioquinol) targeting Abeta amyloid deposition and toxicity in Alzheimer disease: a pilot phase 2 clinical trial," Arch Neurol. 2003 Dec;60(12): 1685-91.
Robert AM, et al, "[Protection of cornea against proteolytic damage. Experimental study of procyanidolic oligomers (PCO) on bovine cornea] J Fr Ophtalmol. 2002 Apr;25(4):351-5
Rotstein NP, et al, "Protective effect of docosahexaenoic acid on oxidative stress-induced apoptosis of retina photoreceptors," Invest Ophthalmol Vis Sci. 2003 May;44(5):2252-9
Rulon LL, et al, "Serum zinc levels and Alzheimer's disease," Biol Trace Elem Res. 2000 Summer;75(1-3): 79-85.
Ryu R, et al, "Depletion of intracellular glutathione mediates zinc-induced cell death in rat primary astrocytes," Exp Brain Res. 2002 Mar;143(2): 257-63.
Sheline CT, et al, "Involvement of poly ADP ribosyl polymerase-1 in acute but not chronic zinc toxicity," Eur J Neurosci. 2003 Skep;18(6): 1402-9.
Siegel, D., "AREDS investigators distort findings," Arch Ophthalm 2002 Jan; 120(1): 100-101.
Sparrow JR, et al, "A2E-epoxides damage DNA in retinal pigment epithelial cells. Vitamin E and other antioxidants inhibit A2E-epoxide formation," J Biol Chem. 2003 May 16;278(20):18207-13
Stoyanovsky DA, et al, "Endogenous ascorbate regenerates vitamin E in the retina directly and in combination with exogenous dihydrolipoic acid," Curr Eye Res. 1995 Mar;14(3):181-9
Suh, SW, et al, "Evidence that synaptically-released zinc contributes to neuronal injury after traumatic brain injury" Brain Research 2000 852:I 268-273
Thomson LR, et al, "Elevated retinal zeaxanthin and prevention of light-induced photoreceptor cell death in quail," Invest Ophthalmol Vis Sci. 2002 Nov;43(11):3538-49.
Trempe C.L., "Zinc and macular degeneration," Arch Ophthalmol. 110: 1517 (1992)
Turner PR, et al, "Roles of amyloid precursor protein and its fragments in regulating neural activity, plasticity and memory," Prog Neurobiol. 2003 May;70(1): 1-32
vanHerpen-Broekmans, W.M., et al, "Serum carotenoids and vitamins in relation to markers of endothelial function and inflammation," Eur J Epidemiol 19(10): 915-21 (2004)
Walrand, S., et al, "In vivo and in vitro evidences that carotenoids could modulate the neutrophil respiratory burst during dietary manipulation," Eur J Nutr May 6: 1-7 (2004)
Yaffe K, et al, "Impact of antioxidants, zinc, and copper on cognition in the elderly: a randomized, controlled trial," Neurology. 2004 Nov 9;63(9):1705-7
Yamakoshi J, et al, "Procyanidin-rich extract from grape seeds prevents cataract formation in hereditary cataractous (ICR/f) rats," J Agric Food Chem. 2002 Aug 14;50(17):4983-8
Yoon HS, et al, "Genrstein produces reduction in growth and induces apoptosis of rat RPE-J cells," Curr Eye Res. 2000 Mar;20(3):215-24
Yuzbasiyan, GV, et al, "The therapeutic use of zinc in macular degeneration, Arch Ophthalmol 1989; 107: 1723-24.

## Claims

1. A nutrient formulation for preventing or treating at least one ocular disorder in mammals, the nutrient formulation in a unit dosage or daily dosage form for oral ingestion wherein said nutrient formulation comprises:
a. 3R,3'R-zeaxanthin, in a daily dosage of at least about 3 milligrams;
b. zinc in a dosage that (i) does not exceed a daily dosage of 40 mg/day of elemental zinc;
c. vitamin C;
d. at least one tocopherol; and
e. at least two additional ocular-active nutrients, each of which is present at a daily dosage that provides synergistic benefits in preventing or treating at least one mammalian ocular disorder when co-administered with said zeaxanthin, wherein said additional ocular-active nutrients are selected from the group consisting of lipoic acid, omega-3 fatty acids, plant-derived flavonoids, anthocyanins and polyphenolics, taurine, carnitine, coenzyme-Q10, carnosine, N-acetyl cysteine, selenium, pyridoxine, riboflavin and folic acid and salts and esters of said compounds,
wherein the nutrient formulation is provided in the form selected from the group consisting of capsules, tablets, liquids, and powders for oral ingestion.

2. A nutrient formulation of claim 1, comprising 3R, 3'R-zeaxanthin, in a daily dosage of at least about 10 milligrams.

3. The nutrient formulation of claim 1, wherein at least one of said additional ocular-active nutrients is present in a stereoisomerically-enriched form, containing a predominance of a stereoisomer of said ocular-active nutrient that is present in normal human diets.

4. The nutrient formulation of claim 1, wherein the at least two additional ocular-active nutrients comprise lipoic acid and omega-3 fatty acids.

5. The nutrient formulation of claim 1, comprising an oily carrier liquid.

6. The nutrient formulation of claim 1 which is intended for therapeutic treatment of at least one known ocular disorder, wherein the at least two additional ocular- active nutrients are present at daily dosages as follows:
a. DHA at a dosage range of 100 to 2000 mg;
b. lipoic acid at a dosage range of 25 to 200 mg;
c. N-acetyl cysteine at a dosage range of 100 to 1000 mg;
d. Co-Q10 at a dosage range of 10 to 100 mg;
e. isoflavones, polyphenols or plant extracts from bilberry, grapes, green tea or soybeans at a dosage range of 10 to 1000 mg;
f. acetyl-L-carnitine at a dosage range of 50 to 500 mg.

7. The nutrient formulation of claim 1 which is intended for preservation of ocular health and prevention of ocular disorders, wherein the at least two additional ocular-active nutrients are present at daily dosages as follows:
a. DHA at a dosage range of 100 to 1000 mg;
b. lipoic acid at a dosage range of 25 to 50 mg;
c. N-acetyl cysteine at a dosage range of 50 to 300 mg;
d. Co-Q10 at a dosage range of 10 to 30 mg;
e. isoflavones, polyphenols or plant extracts from bilberry, grapes, green tea or soybeans at a dosage range of 10 to 1000 mg;
f. acetyl-L-carnitine at a dosage range of 30 to 100 mg.

8. The nutrient formulation of claim 1, where the vitamin C is in the form of ascorbic acid.

9. The nutrient formulation of claim 1, which is present in a form that provides sustained release over a span of at least 3 hours following ingestion.

10. The nutrient formulation of claim 8, wherein the nutrient formulation has been added to and is contained within a food or beverage material.

## Patentansprüche

1. Nährstoffformulierung zum Verhüten oder Behandeln von wenigstens einer Augenkrankheit bei Säugern, wobei die Nährstoffformulierung in einer Dosierungseinheit oder Tagesdosierungsform zur oralen Aufnahme vorliegt, wobei die Nährstoffformulierung umfasst:
a. 3R,3'R-Zeaxanthin, in einer Tagesdosierung von wenigstens ungefähr 3 Milligramm;
b. Zink in einer Dosierung, welche (i) eine Tagesdosierung von 40 mg/Tag von elementarem Zink nicht überschreitet;
c. Vitamin C;
d. wenigstens ein Tocopherol; und
e. wenigstens zwei zusätzliche augenaktive Nährstoffe, von denen jeder in einer Tagesdosierung vorhanden ist, welche synergistische Vorteile beim Verhüten oder Behandeln von wenigstens einer Augenkrankheit bei Säugern bereitstellt, wenn sie zusammen mit dem Zeaxanthin verabreicht werden, wobei die zusätzlichen augenaktiven Nährstoffe ausgewählt sind aus der Gruppe bestehend aus Liponsäure, Omega-3-Fettsäuren, aus Pflanzen gewonnenen Flavonoiden, Anthocyanen und Polyphenolverbindungen, Taurin, Carnitin, Coenzym-Q10, Carnosin, N-Acetyl-cystein, Selen, Pyridoxin, Riboflavin und Folsäure und Salzen und Estern von diesen Verbindungen,
wobei die Nährstoffformulierung in der Form bereitgestellt wird, die ausgewählt ist aus der Gruppe bestehend aus Kapseln, Tabletten, Flüssigkeiten und Pulvern zur oralen Aufnahme.

2. Nährstoffformulierung nach Anspruch 1, umfassend 3R,3'R-Zeaxanthin in einer Tagesdosierung von wenigstens ungefähr 10 Milligramm.

3. Nährstoffformulierung nach Anspruch 1, wobei wenigstens einer von den zusätzlichen augenaktiven Nährstoffen in einer stereoisomer angereicherten Form vorhanden ist, die vorwiegend ein Stereoisomer des augenaktiven Nährstoffs enthält, welches in normalen menschlichen Diäten vorhanden ist.

4. Nährstoffformulierung nach Anspruch 1, wobei die wenigstens zwei zusätzlichen augenaktiven Nährstoffe Liponsäure und Omega-3-Fettsäuren umfassen.

5. Nährstoffformulierung nach Anspruch 1, umfassend eine ölige Trägerflüssigkeit.

6. Nährstoffformulierung nach Anspruch 1, welche für eine therapeutische Behandlung von wenigstens einer bekannten Augenkrankheit bestimmt ist, wobei die wenigstens zwei zusätzlichen augenaktiven Nährstoffe in Tagesdosierungen wie folgt vorhanden sind:
a. DHA in einem Dosierungsbereich von 100 bis 2000 mg;
b. Liponsäure in einem Dosierungsbereich von 25 bis 200 mg;
c. N-Acetylcystein in einem Dosierungsbereich von 100 bis 1000 mg;
d. Co-Q10 in einem Dosierungsbereich von 10 bis 100 mg;
e. Isoflavone, Polyphenole oder Pflanzenextrakte aus Heidelbeere, Weintrauben, grünem Tee oder Sojabohnen in einem Dosierungsbereich von 10 bis 1000 mg;
f. Acetyl-L-carnitin in einem Dosierungsbereich von 50 bis 500 mg.

7. Nährstoffformulierung nach Anspruch 1, welche für die Erhaltung der Augengesundheit und Verhütung von Augenkrankheiten bestimmt ist, wobei die wenigstens zwei zusätzlichen augenaktiven Nährstoffe in Tagesdosierungen wie folgt vorhanden sind:
a. DHA in einem Dosierungsbereich von 100 bis 1000 mg;
b. Liponsäure in einem Dosierungsbereich von 25 bis 50 mg;
c. N-Acetylcystein in einem Dosierungsbereich von 50 bis 300 mg;
d. Co-Q10 in einem Dosierungsbereich von 10 bis 30 mg;
e. Isoflavone, Polyphenole oder Pflanzenextrakte aus Heidelbeere, Weintrauben, grünem Tee oder Sojabohnen in einem Dosierungsbereich von 10 bis 1000 mg;
f. Acetyl-L-carnitin in einem Dosierungsbereich von 30 bis 100 mg.

8. Nährstoffformulierung nach Anspruch 1, wobei das Vitamin C in Form von Ascorbinsäure vorliegt.

9. Nährstoffformulierung nach Anspruch 1, welche in einer Form vorhanden ist, welche eine anhaltende Freisetzung über eine Spanne von wenigstens 3 Stunden nach der Aufnahme bereitstellt.

10. Nährstoffformulierung nach Anspruch 8, wobei die Nährstoffformulierung zu einem Lebensmittel oder Getränkematerial zugegeben wurde und darin enthalten ist.

## Revendications

1. Formule de nutriments pour prévenir ou traiter au moins un trouble oculaire chez les mammifères, la formule de nutriments étant sous une forme posologique unitaire ou posologique quotidienne pour ingestion orale, ladite formule de nutriments comprenant :
a. de la 3R,3'R-zéaxanthine, dans une posologie quotidienne d'au moins environ 3 milligrammes ;
b. du zinc dans une posologie qui (i) ne dépasse pas une posologie quotidienne de 40 mg/jour de zinc élémentaire ;
c. de la vitamine C ;
d. au moins un tocophérol ; et
e. au moins deux nutriments supplémentaires à activité oculaire, chacun d'eux étant présent à une posologie quotidienne qui apporte des bénéfices synergiques en termes de prévention ou de traitement d'au moins un trouble oculaire de mammifère lorsqu'il est co-administré avec ladite zéaxanthine, où lesdits nutriments supplémentaires à activité oculaire sont sélectionnés dans le groupe constitué par l'acide lipoïque, les acides gras oméga-3, les flavonoïdes, anthocyanines et composés polyphénoliques d'origine végétale, la taurine, la carnitine, le coenzyme-Q10, la carnosine, la N-acétylcystéine, le sélénium, la pyridoxine, la riboflavine et l'acide folique et les sels et esters desdits composés,
laquelle formule de nutriments est fournie sous la forme sélectionnée dans le groupe constitué par les capsules, les comprimés, les liquides, et les poudres pour ingestion orale.

2. Formule de nutriments de la revendication 1, comprenant de la 3R,3'R-zéaxanthine, dans une posologie quotidienne d'au moins environ 10 milligrammes.

3. Formule de nutriments de la revendication 1, dans laquelle au moins un desdits nutriments supplémentaires à activité oculaire est présent sous une forme stéréoisomériquement enrichie, contenant une prédominance d'un stéréoisomère dudit nutriment à activité oculaire qui est présent dans les régimes alimentaires normaux de l'être humain.

4. Formule de nutriments de la revendication 1, dans laquelle lesdits au moins deux nutriments supplémentaires à activité oculaire comprennent l'acide lipoïque et des acides gras oméga-3.

5. Formule de nutriments de la revendication 1, comprenant un véhicule liquide huileux.

6. Formule de nutriments de la revendication 1 qui est destinée au traitement thérapeutique d'au moins un trouble oculaire connu, dans laquelle lesdits au moins deux nutriments supplémentaires à activité oculaire sont présents à des posologies quotidiennes comme suit :
a. DHA dans une gamme posologique de 100 à 2000 mg ;
b. acide lipoïque dans une gamme posologique de 25 à 200 mg ;
c. N-acétylcystéine dans une gamme posologique de 100 à 1000 mg ;
d. Co-Q10 dans une gamme posologique de 10 à 100 mg ;
e. isoflavones, polyphénols ou extraits végétaux provenant de myrtille, de raisin, de thé vert ou de soja dans une gamme posologique de 10 à 1000 mg ;
f. acétyl-L-carnitine dans une gamme posologique de 50 à 500 mg.

7. Formule de nutriments de la revendication 1 qui est destinée à la préservation de la santé oculaire et à la prévention de troubles oculaires, dans laquelle lesdits au moins deux nutriments supplémentaires à activité oculaire sont présents à des posologies quotidiennes comme suit :
a. DHA dans une gamme posologique de 100 à 1000 mg ;
b. acide lipoïque dans une gamme posologique de 25 à 50 mg ;
c. N-acétylcystéine dans une gamme posologique de 50 à 300 mg;
d. Co-Q10 dans une gamme posologique de 10 à 30 mg ;
e. isoflavones, polyphénols ou extraits végétaux provenant de myrtille, de raisin, de thé vert ou de soja dans une gamme posologique de 10 à 1000 mg ;
f. acétyl-L-carnitine dans une gamme posologique de 30 à 100 mg.

8. Formule de nutriments de la revendication 1, dans laquelle la vitamine C est sous la forme d'acide ascorbique.

9. Formule de nutriments de la revendication 1, qui est présente sous une forme qui assure une libération prolongée sur une période d'au moins 3 heures après ingestion.

10. Formule de nutriments de la revendication 8, laquelle formule de nutriments a été ajoutée à et est contenue dans une matière pour aliment ou pour boisson.
